# EUROPEAN PATENT APPLICATION

(11) **EP 4 019 541 A1**
(43) Date of publication of application: **29.06.2022**
(21) Application number: 21760356.2
(22) Date of filing: 23.02.2021
(51) Int. Cl.: C07K 16/10, C12N 15/13, C12N 15/63, C07K 1/00, G01N 33/577, G01N 33/569, A61K 39/42, A61P 31/14

(54) **HUMANIZED MONOCLONAL ANTIBODY FOR 2019 NOVEL CORONAVIRUS AND USE THEREOF**

(30) Priority: 24.02.2020 CN 202010114283; 02.03.2020 CN 202010137486; 13.01.2021 CN 202110044541
(71) Applicant: Institute Of Microbiology, Chinese Academy Of Sciences, Beijing 100101 (CN)
(72) Inventor: YAN, Jinghua, Beijing 100101 (CN); SHI, Rui, Beijing 100101 (CN); WANG, Qihui, Beijing 100101 (CN); GAO, Fu, Beijing 100101 (CN); MA, Sufang, Beijing 100101 (CN)
(74) Representative: Elzaburu S.L.P.
(86) International application number: PCT/CN2021/077392
(87) International publication number: WO 2021/169932

(57) **Abstract**

Provided in the present invention are a humanized monoclonal antibody for the 2019 novel coronavirus (2019-nCOV) and the use thereof. The antibody is capable of specifically binding with a receptor binding domain (RBD) of 2019-nCOV and blocking the binding between the RBD of 2019-nCOV and ACE2, and furthermore inhibiting the infection caused by 2019-nCOV.

## Description

### Technical Field

The present invention is in the field of medical technology and specifically relates to a human monoclonal antibody to a novel coronavirus (2019-nCoV, also known as SARS-CoV-2) with high neutralizing activity and the use thereof.

### Background Art

As of 22 Oct. 2020, the number of confirmed cases of novel coronavirus 2019-nCoV-caused diseases (COVID-19) has exceeded 40 million worldwide and the number of deaths has exceeded 1.1 million, posing a major threat to the life and health of the public.
However, there is currently no specific drug for this virus.

Therapeutic antibody drugs play an important role not only in tumor and autoimmune diseases, but also in the treatment of infectious diseases. Currently marketed drugs to treat and prevent viral infections are Synagis to prevent pediatric respiratory syncytial virus (RSV) infection, Trogarzo to treat HIV infection, and Rabishield for the post-exposure prophylaxis of rabies virus. There are also a number of monoclonal antibodies that are at various stages of clinical research (https://clinicaltrials.gov/).

The 2019-nCoV belongs to coronavirus. Severe acute respiratory syndrome coronavirus (SARS-CoV) and Middle East respiratory syndrome coronavirus (MERS-CoV) of the same genus of coronavirus have also caused outbreaks in 2002-2003 and 2012, respectively. According to the World Health Organization (WHO), SARS-CoV causes 8,000 infections and 794 deaths (https://www.who.int/). The number of MERS-CoV infectious virus cases has continued to increase since 2012, with 2,499 confirmed infections and 861 deaths worldwide by the end of 2019. On January 12^{th}, 2020, the World Health Organization formally named the novel coronavirus as "2019 novel coronavirus (2019-nCoV)", and then announced that the new coronavirus (2019-nCoV) was officially classified as severe acute respiratory syndrome coronavirus 2 (SARS-CoV-2) by the International Committee on Taxonomy of Viruses (ICTV) on 11^{th}-12^{th} February 2020. The World Health Organization (WHO) announced that the disease caused by this virus was officially named as "COVID-19" at the Global Research and Innovation Forum held in Geneva on the same day.

To infect a cell, the virus first needs to bind to the host's receptor through the envelope protein. Antibodies, particularly neutralizing active antibodies, block viral infection by binding to envelope proteins, thereby blocking the binding of the virus to cellular receptors. Meanwhile, the antibody binds to the envelope protein, thereby labeling free virus or infected cells, and recruits macrophages or immune cells such as complement and immune molecules through the Fc region of the antibody, thereby eliminating free virus and infected cells. Thus, antibodies that target the receptor binding domain (RBD) not only have the activity to neutralize viral infection, but also act through the Fc region to facilitate clearance of virus and infected cells. Based on studies of other coronaviruses, in particular studies of SARS-CoV and MERS-CoV, an important envelope protein that binds to the receptor is the spike protein (S). S can be further divided into two parts, S1 and S2. The role of S2 is to mediate membrane fusion. Both the N-terminal (NTD) and the C-terminal (CTD) of S1 may be RBDs. Through a study of the 2019-nCoV, the team found that CTD is the RBD of this coronavirus, binding to the receptor ACE2. Thus antibodies that target RBD, and that block S binding to ACE2, may be neutralizing antibodies that inhibit viral infection. The object of the present invention is to identify specific human neutralizing antibodies with protective effect against 2019-nCoV.

### Summary of the Invention

In order to obtain a human neutralizing antibody with protective effect, the present invention firstly selects memory B cells specifically binding to the 2019-nCoV RBD protein from peripheral blood mononuclear cells (PBMCs) of discharged persons cured after 2019-nCoV infection by flow sorting using the 2019-nCoV RBD expressed by insect cells as an antigen, and then performs RT-PCR on the selected single B cells to obtain the variable region sequence and fragment of the antibody, and further links the constant region into an expression vector. After being expressed and purified in mammalian cells, a series of functional tests were performed, including the binding ability to 2019-nCoV RBD protein, the blocking effect of blocking the binding of 2019-nCoV RBD to ACE2, and the neutralizing effect of inhibiting 2019-nCoV infection. Human monoclonal antibodies neutralizing 2019-nCoV infection were obtained, which were named CB6 and GH12, respectively.

Specifically, the present invention is achieved by the following aspects.
(I)
   In one aspect, the present invention provides a human monoclonal antibody or antigen binding fragment thereof that specifically binds to a 2019-nCoV receptor binding domain (RBD) comprising a heavy chain variable region and/or a light chain variable region,
   the heavy chain variable region comprises:
      (I) HCDR1, HCDR2 and HCDR3 with the amino acid sequences shown in SEQ ID NO: 1, SEQ ID NO: 2 and SEQ ID NO: 3, respectively; or HCDR1, HCDR2 and HCDR3 having 1, 2 or 3 amino acid differences with the amino acid sequences shown in SEQ ID NO: 1, SEQ ID NO: 2 and SEQ ID NO: 3, respectively; or
      (II) HCDR1, HCDR2 and HCDR3 with the amino acid sequences shown in SEQ ID NO: 25, SEQ ID NO: 26 and SEQ ID NO: 27, respectively; or HCDR1, HCDR2 and HCDR3 having 1, 2 or 3 amino acid differences with the amino acid sequences shown in SEQ ID NO: 25, SEQ ID NO: 26 and SEQ ID NO: 27, respectively;
   the light chain variable region comprises:
      (I) LCDR1, LCDR2 and LCDR3 with the amino acid sequences shown in SEQ ID NO: 4, SEQ ID NO: 5 and SEQ ID NO: 6, respectively; or LCDR1, LCDR2 and LCDR3 having 1, 2 or 3 amino acid differences with the amino acid sequences shown in SEQ ID NO: 4, SEQ ID NO: 5 and SEQ ID NO: 6, respectively; or
      (II) LCDR1, LCDR2 and LCDR3 with the amino acid sequences shown in SEQ ID NO: 28, SEQ ID NO: 29 and SEQ ID NO: 30, respectively; or LCDR1, LCDR2 and LCDR3 having 1, 2 or 3 amino acid differences with the amino acid sequences shown in SEQ ID NO: 28, SEQ ID NO: 29 and SEQ ID NO: 30, respectively.

In some embodiments, the human monoclonal antibody or antigen binding fragment thereof, wherein the antibody or antigen binding fragment thereof comprises a heavy chain variable region and/or a light chain variable region,
the heavy chain variable region comprises:
   (I) HCDR1, HCDR2 and HCDR3 with the amino acid sequences shown in SEQ ID NO: 1, SEQ ID NO: 2 and SEQ ID NO: 3, respectively; or
   (II) HCDR1, HCDR2 and HCDR3 with the amino acid sequences shown in SEQ ID NO: 25, SEQ ID NO: 26 and SEQ ID NO: 27, respectively;
the light chain variable region comprises:
   (I) LCDR1, LCDR2 and LCDR3 with the amino acid sequences shown in SEQ ID NO: 4, SEQ ID NO: 5 and SEQ ID NO: 6, respectively; or
   (II) LCDR1, LCDR2 and LCDR3 with the amino acid sequences shown in SEQ ID NO: 28, SEQ ID NO: 29 and SEQ ID NO: 30, respectively.

In some embodiments, the human monoclonal antibody or antigen binding fragment thereof, wherein the antibody or antigen binding fragment thereof comprises:
(I) a heavy chain variable region having HCDR1, HCDR2 and HCDR3 with the amino acid sequences shown in SEQ ID NO: 1, SEQ ID NO: 2 and SEQ ID NO: 3, respectively; and a light chain variable region comprising LCDR1, LCDR2 and LCDR3 with the amino acid sequences shown in SEQ ID NO: 4, SEQ ID NO: 5 and SEQ ID NO: 6, respectively; or
(II) a heavy chain variable region having HCDR1, HCDR2 and HCDR3 with the amino acid sequences shown in SEQ ID NO: 25, SEQ ID NO: 26 and SEQ ID NO: 27, respectively; and a light chain variable region having LCDR1, LCDR2 and LCDR3 with the amino acid sequences shown in SEQ ID NO: 28, SEQ ID NO: 29 and SEQ ID NO: 30, respectively.

In some embodiments, the human monoclonal antibody or antigen binding fragment thereof comprises a heavy chain variable region and a light chain variable region:
(I) the heavy chain variable region comprises an amino acid sequence as shown in SEQ ID NO: 7, or an amino acid sequence having at least 95%, 96%, 97%, 98% or 99% sequence identity to the amino acid sequence as shown in SEQ ID NO: 7; and the light chain variable region comprises an amino acid sequence as shown in SEQ ID NO: 8, or an amino acid sequence having at least 95%, 96%, 97%, 98%, or 99% sequence identity to the amino acid sequence as shown in SEQ ID NO: 8; or
(II) the heavy chain variable region comprises an amino acid sequence as shown in SEQ ID NO: 31, or an amino acid sequence having at least 95%, 96%, 97%, 98% or 99% sequence identity to the amino acid sequence as shown in SEQ ID NO: 31; and the light chain variable region comprises an amino acid sequence as shown in SEQ ID NO: 32, or an amino acid sequence having at least 95%, 96%, 97%, 98% or 99% sequence identity to the amino acid sequence as shown in SEQ ID NO: 32.

In some embodiments, the human monoclonal antibody or antigen binding fragment thereof comprises:
(I) a heavy chain variable region having an amino acid sequence as shown in SEQ ID NO: 7 and a light chain variable region having an amino acid sequence as shown in SEQ ID NO: 8;
(II) a heavy chain variable region having an amino acid sequence as shown in SEQ ID NO: 31 and a light chain variable region having an amino acid sequence as shown in SEQ ID NO: 32. In some embodiments, the human monoclonal antibody or antigen binding fragment thereof, wherein the antibody comprises a heavy chain and a light chain:
   (I) the heavy chain comprises an amino acid sequence as shown in SEQ ID NO: 22, or an amino acid sequence having at least 90%, 92%, 94%, 95%, 96%, 97%, 98%, or 99% sequence identity to the amino acid sequence as shown in SEQ ID NO: 22; and the light chain comprises an amino acid sequence as shown in SEQ ID NO: 23, or an amino acid sequence having at least 90%, 92%, 94%, 95%, 96%, 97%, 98%, or 99% sequence identity to the amino acid sequence as shown in SEQ ID NO: 23; or
   (II) the heavy chain comprises an amino acid sequence as shown in SEQ ID NO: 33, or an amino acid sequence having at least 90%, 92%, 94%, 95%, 96%, 97%, 98%, or 99% sequence identity to the amino acid sequence as shown in SEQ ID NO: 33; and the light chain comprises an amino acid sequence as shown in SEQ ID NO: 34, or an amino acid sequence having at least 90%, 92%, 94%, 95%, 96%, 97%, 98%, or 99% sequence identity to the amino acid sequence as shown in SEQ ID NO: 34.

In some embodiments, the human monoclonal antibody or antigen binding fragment thereof, wherein the antibody comprises:
(I) a heavy chain having an amino acid sequence as shown in SEQ ID NO: 22, and a light chain having an amino acid sequence as shown in SEQ ID NO: 23; or
(II) a heavy chain having an amino acid sequence as shown in SEQ ID NO: 33, and a light chain having an amino acid sequence as shown in SEQ ID NO: 34.

In some embodiments, the human monoclonal antibody or antigen binding fragment thereof, wherein the antigen binding fragment is selected from Fab, Fab', Fab'-SH, Fv, scFv, F(ab')2, diabody.

In some embodiments, the invention provides a polypeptide comprising an amino acid sequence selected from the group consisting of SEQ ID NOs: 7, 8, 22 or 23.

In some embodiments, the invention provides a polypeptide comprising an amino acid sequence selected from the group consisting of SEQ ID NOs: 31, 32, 33 or 34.

In some embodiments, the invention provides a polypeptide comprising a sequence selected from SEQ ID NOs: 31, 32, 33 or 34, wherein the polypeptide is a portion of a human monoclonal antibody that specifically binds 2019-nCoV RBD, and
when the polypeptide comprises SEQ ID NO: 31, the human monoclonal antibody further comprises the polypeptide as shown in SEQ ID NO: 32;
when the polypeptide comprises SEQ ID NO: 32, the human monoclonal antibody further comprises the polypeptide as shown in SEQ ID NO: 31;
when the polypeptide comprises SEQ ID NO: 33, the human monoclonal antibody further comprises the polypeptide as shown in SEQ ID NO: 34; or
when the polypeptide comprises SEQ ID NO: 34, the human monoclonal antibody further comprises the polypeptide as shown in SEQ ID NO: 33.

In yet another aspect, the invention provides a polynucleotide encoding the human monoclonal antibody or antigen binding fragment thereof as described herein or the polypeptide as described herein.

In a further aspect, the invention provides an expression vector comprising the polynucleotide as described herein, preferably the vector is a eukaryotic expression vector.

In yet another aspect, the invention provides a host cell comprising a polynucleotide or expression vector as described herein, preferably the host cell is a eukaryotic cell, more preferably a mammalian cell.

In yet another aspect, the present invention provides a method of producing the human monoclonal antibody or antigen binding fragment thereof or the polypeptide as described herein, the method comprising expressing the antibody or antigen binding fragment thereof or the polypeptide in a host cell as described herein under conditions suitable for expression of the antibody or antigen binding fragment thereof or the polypeptide, and recovering the expressed antibody or antigen binding fragment thereof from the host cell.

In yet another aspect, the invention provides a pharmaceutical composition comprising the human monoclonal antibody or antigen binding fragment thereof as described herein, or the polypeptide, the polynucleotide, the expression vector and/or the host cell, and a pharmaceutically acceptable carrier.

In yet another aspect, the present invention provides the use of the human monoclonal antibody or antigen binding fragment thereof, the polypeptide or the pharmaceutical composition as described herein in the manufacture of a medicament for the treatment and/or prevention of a 2019-nCoV infection.

In yet another aspect, the present invention provides a method of treating and/or preventing a 2019-nCoV infection comprising administering to a subject in need thereof the human monoclonal antibody or antigen binding fragment thereof, the polypeptide, the polynucleotide, the expression vector, the host cell and/or the pharmaceutical composition as described herein. In yet another aspect, the invention provides a kit comprising the antibody or antigen binding fragment thereof as described herein, the polypeptide, the polynucleotide, the expression vector, the host cell and/or the pharmaceutical composition as described herein.

In some embodiments, the invention provides the use of the kit in the manufacture of a medicament for diagnosing a 2019-nCoV infection.

In yet another aspect, the present invention provides a method for detecting the presence of a 2019-nCoV in a sample using the antibody or antigen binding fragment thereof or the polypeptide as described herein.

In one aspect, the present invention provides a human monoclonal antibody or antigen binding fragment thereof that specifically binds to 2019-nCoV RBD,
the CDRs of the complementarity determining region of the VH chain thereof have an amino acid sequence selected from the group consisting of:
   CDR1 as shown in SEQ ID NO: 1,
   CDR2 as shown in SEQ ID NO: 2, and
   CDR3 as shown in SEQ ID NO: 3;
the CDRs of the complementarity determining region of the VL chain thereof have an amino acid sequence selected from the group consisting of:
   CDR1 as shown in SEQ ID NO: 4,
   CDR2 as shown in SEQ ID NO: 5, and
   CDR3 as shown in SEQ ID NO: 6.

In one aspect, the present invention provides a human monoclonal antibody or antigen binding fragment thereof that specifically binds to 2019-nCoV RBD,
the CDRs of the complementarity determining region of the VH chain thereof have an amino acid sequence selected from the group consisting of:
   CDR1 as shown in SEQ ID NO: 25,
   CDR2 as shown in SEQ ID NO: 26, and
   CDR3 as shown in SEQ ID NO: 27;
the CDRs of the complementarity determining region of the VL chain thereof have an amino acid sequence selected from the group consisting of:
   CDR1 as shown in SEQ ID NO: 28,
   CDR2 as shown in SEQ ID NO: 29, and
   CDR3 as shown in SEQ ID NO: 30.

In one embodiment, the human monoclonal antibody or antigen binding fragment thereof comprises:
a heavy chain variable region as shown in SEQ ID NO: 7, and
a light chain variable region as shown in SEQ ID NO: 8.

In one embodiment, the human monoclonal antibody or antigen binding fragment thereof comprises:
a heavy chain variable region as shown in SEQ ID NO: 31, and
a light chain variable region as shown in SEQ ID NO: 32.

In one embodiment, a human monoclonal antibody or antigen binding fragment thereof comprises:
a heavy chain as shown in SEQ ID NO: 22, and
a light chain as shown in SEQ ID NO: 23.

In one embodiment, a human monoclonal antibody or antigen binding fragment thereof comprises:
a heavy chain as shown in SEQ ID NO: 33, and
a light chain as shown in SEQ ID NO: 34.

In one embodiment, wherein the antigen binding fragment is selected from Fab, Fab', Fab'-SH, Fv, scFv, F(ab')2, diabody.

In another aspect, the invention provides a polypeptide comprising a sequence selected from SEQ ID NOs: 7, 8, 22 or 23.

In another aspect, the invention provides a polypeptide comprising a sequence selected from SEQ ID NOs: 31, 32, 33 or 34, wherein the polypeptide is part of a human monoclonal antibody that specifically binds COVID-19 RBD, and
when the polypeptide comprises SEQ ID NO: 31, the human monoclonal antibody further comprises the polypeptide as shown in SEQ ID NO: 32;
when the polypeptide comprises SEQ ID NO: 32, the human monoclonal antibody further comprises the polypeptide as shown in SEQ ID NO: 31;
when the polypeptide comprises SEQ ID NO: 33, the human monoclonal antibody further comprises the polypeptide as shown in SEQ ID NO: 34; or
when the polypeptide comprises SEQ ID NO: 34, the human monoclonal antibody further comprises the polypeptide as shown in SEQ ID NO: 33.

In another aspect, the invention provides a polynucleotide encoding any one of the foregoing human monoclonal antibodies or antigen binding fragments thereof, or any one of the foregoing polypeptides.

In yet another aspect, the present invention provides an expression vector comprising the polynucleotide described above.

In yet another aspect, the present invention provides a host cell comprising the expression vector described above.

In yet another aspect, the invention provides a pharmaceutical composition comprising any one of the foregoing human monoclonal antibodies or antigen binding fragments thereof and a pharmaceutically acceptable carrier.

In yet another aspect, the invention provides the use of any one of the above-described human monoclonal antibodies or antigen binding fragments thereof in the manufacture of a medicament for treating a 2019-nCoV infection.

The human monoclonal antibodies or antigen binding fragments thereof disclosed herein also specifically bind to coronaviruses such as SARS-CoV RBD or MERS-CoV RBD.

All documents mentioned in this specification are hereby incorporated by reference in its entirety.

### Definitions

The practice of the present invention will employ, unless otherwise indicated, conventional techniques of molecular biology (including recombinant techniques), microbiology, cell biology, biochemistry and immunology, which are within the skill of the art.

In order that the present invention may be more readily understood, certain scientific and technical terms are specifically defined as follows. Unless otherwise defined in other parts herein, all technical and scientific terms used herein have the same meaning as commonly understood by a person having ordinary skill in the art to which this invention belongs. For definitions and terms of the art, one skilled in the art may refer specifically to Current Protocols in Molecular Biology (Ausubel). Abbreviations for amino acid residues are standard 3-letter and/or 1-letter codes used in the art to refer to one of the 20 commonly used L-amino acids. As used herein, including the claims, the singular forms "a", "an", and "the" include the corresponding plural unless the context clearly dictates otherwise.

The term "about", when used in conjunction with a numerical value, is intended to encompass numerical values within a range having a lower limit that is 5% less than the specified numerical value, and an upper limit that is 5% greater than the specified numerical value, including, but not limited to, ± 5%, ± 2%, ± 1%, and ± 0.1%, as such variations are appropriate to perform the disclosed methods.

The term "and/or" is understood to mean any one of the alternatives or a combination of any two or more of the alternatives.

As used herein, the term "or" should be understood to have the same meaning as "and/or" as defined above. For example, when separating items in a list, "or" or "and/or" should be interpreted as inclusive, i.e. including at least one, but also including more than one, of the number or list of elements, and, optionally, additional unlisted items. Only if terms clearly indicated to the contrary, such as "only one" or "indeed one", or "consisting of" used in the claims, will refer to only one number or one element of a list.

The term "percent (%) amino acid sequence identity" or simply "identity" is defined as the percentage of amino acid residues in a candidate amino acid sequence that are identical to the amino acid residues in a reference amino acid sequence after aligning to the amino acid sequences (and introducing gaps, if necessary) to obtain the maximum percent sequence identity without considering any conservative substitutions as part of the sequence identity. Sequence alignments can be performed using various methods known in the art to determine percent amino acid sequence identity, for example, using publicly available computer software such as BLAST, BLAST-2, ALIGN, or MEGALIGN (DNASTAR) software. One skilled in the art can determine the appropriate parameters for measuring the alignment, including any algorithm required to obtain the maximum alignment over the full length of the sequences to being compared.

The term "antibody" refers to any form of antibody having the desired biological activity. Thus, it is used in its broadest sense and specifically includes, but is not limited to, monoclonal antibodies (including full length monoclonal antibodies), polyclonal antibodies, multispecific antibodies (e. g. bispecific antibodies), humanized antibodies, whole human antibodies, chimeric antibodies, and camelized single domain antibodies.

The term "monoclonal antibody" refers to an antibody obtained from a substantially homogeneous population of antibodies, i.e. the individual antibody making up the population is identical except for possible naturally occurring mutations that may be present in minor amounts. Monoclonal antibodies are highly specific, being directed against a single epitope. In contrast, conventional (polyclonal) antibody preparations typically include a large number of antibodies directed against (or specific for) different epitopes. The modifier "monoclonal" indicates the character of the antibody as being obtained from a substantially homogeneous population of antibodies, and is not to be construed as requiring production of the antibody by any particular method.

"Antigen binding fragment" refers to antigen binding fragments of antibodies and antibody analogs that generally include at least a portion of the antigen-binding region or variable region of the parent antibody, e. g. one or more CDRs. A fragment of an antibody retains at least some of the binding specificity of the parent antibody. Antigen binding fragments include those selected from the group consisting of Fab, Fab', Fab'-SH, Fv, scFv, F(ab')₂, diabody, CDR-containing peptides, and the like.

A "Fab fragment" consists of the CH1 and variable regions of one light chain and one heavy chain.

A "Fc" region contains two heavy chain fragments comprising the CH2 and CH3 domains of the antibody. The two heavy chain fragments are held together by two or more disulfide bonds and by the hydrophobic action of the CH3 domain.

A "Fab' fragment" comprises a light chain and a portion of a heavy chain comprising portions of a VH domain, a CH1 domain, and a constant region between the CH1 and CH2 domains, an interchain disulfide bond being formed between two heavy chains of two Fab' fragments to form a F(ab')₂ molecule.

A "F(ab')₂ fragment" comprises two light chains and two portions of a heavy chain comprising portions of a VH domain, a CH1 domain, and a constant region between the CH1 and CH2 domains, thereby forming an interchain disulfide bond between the two heavy chains. Thus, a F(ab')₂ fragment consists of two Fab' fragments held together by a disulfide bond between two heavy chains.

A "Fv region" comprises variable regions from both heavy and light chains, but lacks constant regions.

A "single chain Fv antibody" (scFv antibody) refers to an antigen binding fragment comprising the VH and VL domains of an antibody, which domains are comprised in a single polypeptide chain. Generally, scFv polypeptides comprise a polypeptide linker between the VH and VL domains that enables the scFv to form the desired structure for antigen binding.

A "diabody" is a small antigen binding fragment having two antigen-binding sites. The fragment comprises a heavy chain variable domain (VH) linked to a light chain variable domain (VL) in the same polypeptide chain (VH-VL or VL-VH). By using a linker that is short and not enable to pair between two domains of the same chain, the domains pair with complementary domains of the other chain and form two antigen binding sites.

"Specific" binding, when referring to a ligand/receptor, antibody/antigen or other binding pair, refers to determining the presence or absence of a binding reaction of a protein, such as a monoclonal antibody of the invention, with a 2019-nCoV RBD protein in a heterogeneous population of proteins and/or other biological agents. Thus, under the specified conditions, a particular ligand/antigen binds to a particular receptor/antibody and does not bind in a significant amount to other proteins present in the sample.

"Affinity" or "binding affinity" refers to the intrinsic binding affinity that reflects the interaction between members of a binding pair. The affinity of a molecule X for its partner Y can generally be represented by the equilibrium dissociation constant (KD), which is the ratio of the dissociation rate constant and the binding rate constant (kdis and kon, respectively). Affinity can be measured by common methods known in the art. One particular method for measuring affinity is the ForteBio kinetic binding assay herein. The term "does not bind" to a protein or cell means that it does not bind to a protein or cell, or does not bind to it with high affinity, i.e. the K_{D} of the binding protein or cell is 1.0 × 10⁻⁶ M or more, more preferably 1.0 × 10⁻⁵ M or more, more preferably 1.0 × 10⁻⁴ M or more, 1.0 × 10⁻³ M or more, more preferably 1.0 × 10⁻² M or more.

The term "high affinity" for IgG antibodies means a K_{D} for the antigen of 1.0 × 10⁻⁶ M or less, preferably 5.0 × 10⁻⁸ M or less, more preferably 1.0 × 10⁻⁸ M or less, 5.0 × 10⁻⁹ M or less, more preferably 1.0 × 10⁻⁹ M or less. For other antibody subtypes, "high affinity" binding may vary. For example, "high affinity" binding of an IgM subtype refers to a K_{D} of 10⁻⁶ M or less, preferably 10⁻⁷ M or less, more preferably 10⁻⁸ M or less.

The term "nucleic acid" or "polynucleotide" refers to deoxyribonucleic acid (DNA) or ribonucleic acid (RNA) and polymers thereof in single-stranded or double-stranded form. Unless specifically limited, the term includes nucleic acids containing analogs of known natural nucleotides that have similar binding properties as reference nucleic acids and are metabolized in a manner similar to naturally occurring nucleotides (see, U.S. Patent No. 8,278,036 of Kariko et al. which discloses mRNA molecules in which uridine is replaced by pseudouridine, methods of synthesizing the mRNA molecules, and methods for delivering therapeutic proteins in vivo). Unless otherwise indicated, a particular nucleic acid sequence also implicitly includes conservatively modified variants (e. g. degenerate codon substitutions), alleles, orthologs, SNPs, and complementary sequences thereof, as well as the sequence explicitly indicated. In particular, degenerate codon substitutions may be achieved by generating sequences in which the third position of one or more selected (or all) codons is replaced by mixed base and/or deoxyinosine residues (Batzer et al., Nucleic Acid Res., 19: 5081 (1991); ohtsuka et al., J. Biol. Chem., 260: 2605-2608 (1985); and Rossolini et al., Mol. Cell. Probes, 8: 91-98 (1994)).

"Construct" refers to any recombinant polynucleotide molecule (such as a plasmid, cosmid, virus, autonomously replicating polynucleotide molecule, phage or linear or circular single-stranded or double-stranded DNA or RNA polynucleotide molecule), derived from any source, capable of integrating with the genome or autonomously replicating, that constitutes a polynucleotide molecule in which one or more polynucleotide molecules have been functionally linked (i.e. operably linked). A recombinant construct will typically comprise a polynucleotide of the invention operably linked to transcription initiation regulatory sequences that direct transcription of the polynucleotide in a host cell. Both heterologous and non-heterologous (i.e. endogenous) promoter may be used to guide the expression of the nucleic acids of the present invention.

"Vector" refers to any recombinant polynucleotide construct that can be used for transformation purposes (i.e. the introduction of heterologous DNA into a host cell). One type of vector is a "plasmid", which refers to a circular double-stranded DNA loop into which additional DNA segments can be linked. Another type of vector is a viral vector in which additional DNA segments can be linked into the viral genome. Certain vectors are capable of autonomous replication in a host cell into which they are introduced (e. g. bacterial vectors having a bacterial origin of replication and episomal mammalian vectors). Other vectors (e.g. non-episomal mammalian vectors) are integrated into the genome of a host cell upon introduction into the host cell, and thereby are replicated along with the host genome. In addition, certain vectors are capable of guiding the expression of an operably linked gene. Such vectors are referred to herein as "expression vectors".

As used herein, the term "expression vector" refers to a nucleic acid molecule capable of replicating and expressing a gene of interest when transformed, transfected or transduced into a host cell. Expression vectors contain one or more phenotypic selectable markers and an origin of replication to ensure maintenance of the vector and to provide amplification in the host if desired.

The present invention also provides pharmaceutical compositions comprising a human high neutralizing activity monoclonal antibody or antigen binding fragment thereof that specifically binds 2019-nCoV RBD of the present invention. To prepare a pharmaceutical composition, the antibody or antigen binding fragment thereof can be formulated into various desired dosage forms by mixing with a pharmaceutically acceptable carrier or excipient. As types of the dosage form of the pharmaceutical composition of the present invention, for example, tablets, powders, pills, pulvis, granules, fine granules, soft/hard capsules, film coatings, pellets, sublingual tablets, ointments and the like can be cited as oral agents, and as non-oral agents, injections, suppositories, transdermal agents, ointments, plasters, external liquid preparations and the like can be cited, and those skilled in the art can select an appropriate dosage form according to a route of administration and a subject of administration and the like.

The amount of the active ingredient of the pharmaceutical composition of the present invention to be administered varies depending on the subject to be administered, the organ of the subject, the symptom, the method of administration, etc., and may be determined according to the judgment of a physician considering the type of dosage form, the method of administration, the age and weight of the patient, the symptom of the patient, etc.

### Beneficial effects of the present invention:

The present invention achieves high neutralizing activity antibodies of human origin: CB6 and GH12. The above antibodies are human antibodies with neutralizing 2019-nCoV infection. The binding constant of the CB6 antibody to the 2019-nCoV was 8.15E-10 M and the affinity of the GH12 antibody to the 2019-nCoV RBD was 5.87E-09 M. Human high neutralizing activity antibodies CB6 and GH12 can effectively block the binding of 2019-nCoV RBD to hACE2, and inhibit the neutralizing activity of 2019-nCoV pseudovirus infection. The CB6 and GH12 of the present invention have clinical utility in the treatment and prevention of 2019-nCoV infection.

### Brief Description of the Drawings

FIG. 1: gel filtration chromatography and SDS-PAGE identification of 2019-nCoV RBD.
FIG. 2: 2a: molecular sieve chromatography and SDS-PAGE identification of CB6 antibody; 2b: molecular sieve chromatography and SDS-PAGE identification of GH12 antibody.
FIG. 3: 3a: kinetic profile of CB6 antibody binding to 2019-nCoV RBD; 3b: kinetic profile of GH12 antibody binding to 2019-nCoV RBD.
FIG. 4: 4a: CB6 antibody blocks binding of 2019-nCoV RBD to HEK293T-hACE2; 4b: GH12 antibody blocks binding of 2019-nCoV RBD to HEK293T-hACE2.
FIG. 5: 5a: effect of CB6 antibody to neutralize VSV-2019-nCoV infection; 5b: effect of GH12 antibody to neutralize VSV-2019-nCoV infection.
FIG. 6: neutralization of CB6 antibodies on SARS-CoV-2 live virus.
FIG. 7: body temperature and weight change of experimental animals: 7a: temperature change of experimental animals; 7b: body weight changes of experimental animals.
FIG. 8: changes in virus load of throat swab, nasal swab and anal swab of experimental animals: 8a: throat swab viral load: the virus load of throat swab reflects that the virus can be effectively amplified in animals, but after antibody treatment, the virus load greatly decreases or cannot be detected basically; 8b: nasal swab viral load: the viral load in nasal swabs is low, all near or below the limit of detection; 8c: anal swab viral load: the viral load is low, all near or below the limit of detection.
   L. O. D (limit of detection): 200 copies/ml.
FIG. 9: imaging analysis of experimental animals: 9a: radiographic observation of animals in control group, (top): C1, (middle): C2, (bottom): C3; 9b: imaging observation of animals in prevention group (top): PA1, (middle): PA2, (bottom): PA3; 9c: imaging observations of animals in treatment group (top): AC1, (medium): AC2, (bottom) AC3.
FIG. 10: analysis of tracheal, bronchial and pulmonary viral load of experimental animals: 10a: day 5 after challenge; 10b: day 6 after challenge; 10c: day 7 after challenge.
   L. O. D (limit of detection): limit of detection, 1000 copies/g.

### Detailed Description of the Invention

In order to make the objects, aspects and advantages of the present invention more apparent, a more particular description of the invention will be rendered in detail in the following in combination with specific embodiments and with reference to the drawings.

### Example 1: expression and purification of 2019-nCoV RBD

To the 3' end of the coding region of the 2019-nCoV RBD protein (amino acid sequence as shown in SEQ ID NO: 9) was linked the coding sequence of six histidine tags (hexa-His-tag) and a translation terminator codon, and constructed into pFastBac1 vector (purchased from Invitrogen) by linking EcoRI and Xhol. The linking product was then transformed into DH10Bac competent cells (purchased from Tiangen) for baculovirus recombination. Recombinant baculovirus was extracted, transfected into sf9 cells (purchased from Invitrogen) for packaging of baculovirus, amplified, added to Hi5 cells (purchased from Invitrogen) for expression of the 2019-nCoV RBD protein.

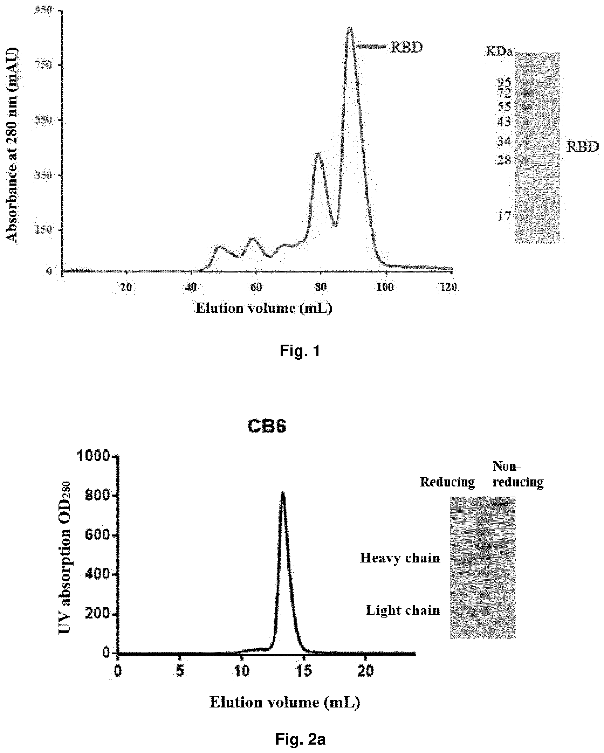

After purification by nickel ion affinity chromatography (HisTrap TMHP (GE)) and gel filtration chromatography (SuperoseTM 6 Increase 10/300 GL (GE)), relatively pure target protein can be obtained from the cell culture solution containing the target protein. SDS-PAGE identified a size of 30 KD and the results are shown in FIG. 1.

### Example 2: isolation of 2019-nCoV RBD protein specific memory B cells

With the informed consent of the discharged person recovering after 2019-nCoV infection, 15 mL of blood was collected and PBMCs were isolated. Isolated PBMCs were incubated at a density of 10⁷/mL with 2019-nCoV RBD protein at a final concentration of 400 nM for binding for half an hour on ice, then washed twice with PBS and then incubated with the following antibodies (all purchased from BD): anti-human CD3/PE-Cy5, anti-human CD16/PE-Cy5, anti-human CD235a/PE-Cy5, anti-human CD19/APC-Cy7, anti-human CD27/Pacific Blue, anti-human CD38/APC, anti-human IgG/FITC, and anti-His/PE. After half an hour of incubation on ice, PBMCs were washed twice with PBS.

The PBS-washed PBMCs were sorted by FACSAria III, and cells of PE-Cy5-APC-APC-Cy7 + Pacific Blue + FITC + PE + (i.e. B cells) were collected directly into 96-well plates, 1 cell/well.

### Example 3: single B cell PCR, sequence analysis and human antibody design

According to the method described in Qihui Wang et al. Molecular determinants of human neutralizing antibodies isolated from a patient infected with Zika virus, science Translational Medicine, vol. 8, No. 369, Dec. 2016, the B cells obtained in Example 2 were reverse transcribed by Superscript III reverse transcriptase (Invitrogen) with reverse transcription primers as shown in Table 1 and reacted at 55°C for 60 min.

**Table 1. Reverse transcription reaction primers**

| Primer | Sequence No. 5 | '-3' sequence |
|---|---|---|
| IgM-RT | SEQ ID NO: 10 | ATG GAG TCG GGA AGG AAG TC |
| IgD-RT | SEQ ID NO: 11 | TCA CGG ACG TTG GGT GGT A |
| IgE-RT | SEQ ID NO: 12 | TCA CGG AGG TGG CAT TGG A |
| IgA1-RT | SEQ ID NO: 13 | CAG GCG ATG ACC ACG TTC C |
| IgA2-RT | SEQ ID NO: 14 | CAT GCG ACG ACC ACG TTC C |
| IgG-RT | SEQ ID NO: 15 | AGG TGT GCA CGC CGC TGG TC |
| Cκ-new RT | SEQ ID NO: 16 | GCA GGC ACA CAA CAG AGG CA |
| Cλ-new-ext | SEQ ID NO: 17 | AGG CCA CTG TCA CAG CT |

### 3.1 Human antibody CB6

Using the above reverse transcription products as templates, antibody variable region sequences were amplified by PCR (PCRa) using HotStar Tap Plus enzyme (QIAgen). The corresponding primers are designed and the reaction conditions are as follows: 95°C for 5 min; 95°C for 30 s, 55°C (heavy chain) for 30 s, 72°C for 90 s, 35 cycles; 72°C for 7min. This product was used as a template for another round of PCR (PCRb) under the following conditions: 95°C for 5 min; 95°C for 30 s, 58°C (heavy chain)/60°C (κ chain) for 30 s, 72°C for 90 s, 35 cycles; 72°C for 7min, to obtain the PCR products.

The PCR products were separated by 1.2% agarose gel electrophoresis. Gels with band sizes between 400 and 500 bp were recovered and sent to sequencing. Sequencing results were analyzed using IMGT online software.

The correct variable region sequence from the analysis were linked with the corresponding heavy/kappa chain constant regions by bridge-PCR and cloned into the expression vector pCAGGS (purchased from Addgene). The heavy chain was linked with EcoRI and Xhol, and the κ chain was linked with Sacl and Xhol. B cell sequencing and expression plasmid construction is as follows:
the human antibody design strategy is as follows:
heavy chain: CMV promoter-EcoR I-leader sequence-heavy chain variable region-CH-Xho I;
light chain (κ): CMV promoter-Sac I-leader sequence-light chain variable region-CL (κ)-Xho I; wherein, the amino acid sequence of the leader sequence is shown in SEQ ID NO: 18, the amino acid sequence of CH is shown in SEQ ID NO: 19, and the amino acid sequence of CL is shown in SEQ ID NO: 20. By sequence determination, the sequence of an antibody named CB6 was obtained.

Wherein the heavy chain variable region sequence of CB6 is shown in SEQ ID NO: 7, the light chain variable region sequence is shown in SEQ ID NO: 8, the heavy chain sequence is shown in SEQ ID NO: 22, and the light chain sequence is shown in SEQ ID NO: 23.

The heavy chain variable region of CB6 has HCDR1, HCDR2 and HCDR3 as shown in SEQ ID NO: 1, SEQ ID NO: 2 and SEQ ID NO: 3, respectively, and the light chain variable region has an amino acid sequence of LCDR1, LCDR2 and LCDR3 as shown in SEQ ID NO: 4, SEQ ID NO: 5 and SEQ ID NO: 6, respectively. Wherein the sequence identity of the CB6 antibody to the germline gene is compared as follows:

**Table 2. Comparison of CB6 antibody heavy chain and germline genes**

| | V-H allele | D-H allele | J-H allele | Consistency (V-H) |
|---|---|---|---|---|
| CB6 | IGHV3-66*01 | IGHD3-10*02 | IGHJ4*02 | 99.00% |

**Table 3. Comparison of CB6 antibody light chain with germline genes**

| | V-L allele | J-L allele | Consistency (V-L) |
|---|---|---|---|
| CB6 | IGKV1-39*01 | IGKJ2*01 | 99.60% |

### 3.2 Human antibody GH12

Using the above reverse transcription products as templates, antibody variable region sequences were amplified by PCR (PCRa) using HotStar Tap Plus enzyme (QIAgen). The corresponding primers are designed and the reaction conditions are as follows: 95°C for 5 min; 95°C for 30 s, 55°C (heavy chain)/50°C (lambda-chain) for 30 s, 72°C for 90 s, 35 cycles; 72°C for 7min. This product was used as a template for another round of PCR (PCRb) under the following conditions: 95°C for 5 min; 95°C for 30 s, 58°C (heavy chain)/64°C (lambda-chain) for 30 s, 72°C for 90 s, 35 cycles; 72°C for 7min, to obtain the PCR products.

The PCR products were separated by 1.2% agarose gel electrophoresis. Gels with band sizes between 400 and 500 bp were recovered and sent to sequencing. Sequencing results were analyzed using IMGT online software.

The correct variable region sequence from the analysis were linked with the corresponding heavy/lambda chain constant regions by bridge-PCR and cloned into the expression vector pCAGGS (purchased from Addgene). Wherein the heavy chain is linked to the lambda chain with EcoRI and Xhol. B cell sequencing and expression plasmid construction is as follows:
the human antibody design strategy is as follows:
heavy chain: CMV promoter-EcoR I-leader sequence-heavy chain variable region-CH-Xho I;
Light chain (λ): CMV promoter-EcoR I-leader sequence-light chain variable region-CL (A)-Xho I; wherein, the amino acid sequence of the leader sequence is shown in SEQ ID NO: 18, the amino acid sequence of CH is shown in SEQ ID NO: 19, and the amino acid sequence of CL is shown in SEQ ID NO: 24. By sequence determination, the sequence of an antibody named GH12 was obtained.

Wherein the heavy chain variable region sequence of GH12 is shown in SEQ ID NO: 31, the light chain variable region sequence is shown in SEQ ID NO: 32, the heavy chain sequence is shown in SEQ ID NO: 33, and the light chain sequence is shown in SEQ ID NO: 34.

The heavy chain variable region of GH12 has HCDR1, HCDR2 and HCDR3 as shown in SEQ ID NO: 25, SEQ ID NO: 26 and SEQ ID NO: 27, respectively, and the light chain variable region has an amino acid sequence of LCDR1, LCDR2 and LCDR3 as shown in SEQ ID NO: 28, SEQ ID NO: 29 and SEQ ID NO: 30, respectively.

Wherein the sequence identity of the GH12 antibody to the germline gene is compared as follows:

**Table 4. Comparison of GH12 antibody heavy chain and germline genes**

| | V-H allele | D-H allele | J-H allele | Consistency (V-H) |
|---|---|---|---|---|
| GH12 | IGHV3-7*01 | IGHD3-10*01 | IGHJ3*02 | 99.70% |

**Table 5. Comparison of GH12 antibody light chain with germline genes**

| | V-L allele | J-L allele | Consistency (V-L) |
|---|---|---|---|
| GH12 | IGLV6-57*02 | IGLJ3*02 | 100.00% |

### Example 4: Expression of antibodies

293T cells were cultured in DMEM containing 10% FBS. 293T was co-transfected with a plasmid containing genes encoding the light and heavy chains of the specific antibody obtained in Example 3. After 4-6 hours of transfection, the cell culture medium was changed to serum-free DMEM, and the culture was continued for 3 days. After the supernatant was collected, DMEM was added again, and the culture was continued for 4 days, and the supernatant was collected.

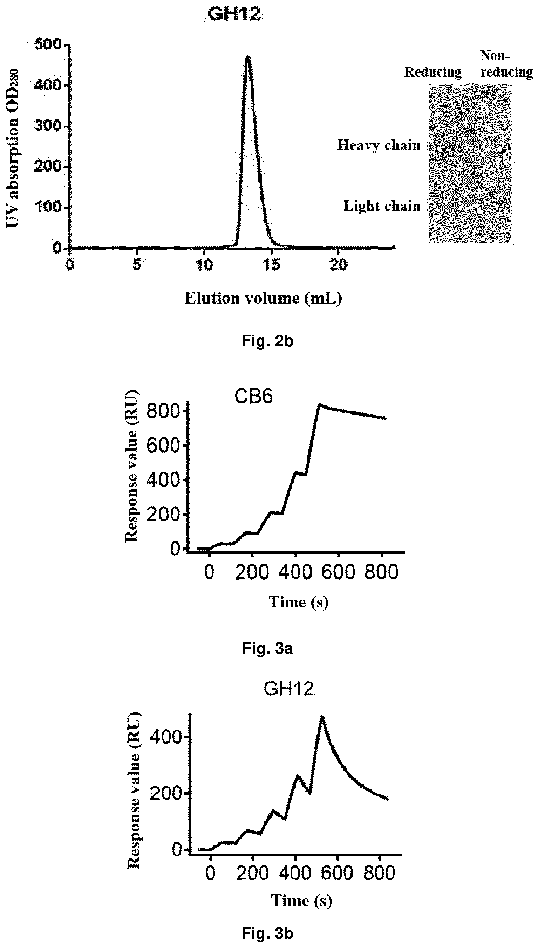

The collected supernatant was centrifuged at 5000 rpm for 30 min, mixed with an equal volume of buffer containing 20 mM sodium phosphate (pH 8.0), filtered through a 0.22 µm filter membrane, and combined with a protein A pre-packed column (5 mL, GE Healthcare). Bound proteins were eluted with 10 mM glycine (pH 3.0). The protein was collected and concentrated and subjected to molecular sieve chromatography. Peaks of interest were determined by SDS-PAGE (reducing and non-reducing) and the results are shown in FIG. 2a (CB6 antibody) and FIG. 2b (GH12 antibody). Purified CB6 and GH12 antibodies were obtained.

### Example 5: Surface plasmon resonance detection of binding capacity of antibody to 2019-nCoV RBD

Surface plasmon resonance analysis was performed using Biacore 8K (Biacore Inc.). The specific steps are as follows:
protein A chip (purchased from GE Healthcare) was used, the purified antibody obtained in Example 4 was immobilized on the chip by affinity of protein A to Fc of the antibody in an amount of about 5000 RU, and 2019-nCoV RBD protein was diluted with 10 mM HEPES, 150 mM NaCl, pH 7.4 solution in double solution, and loaded one by one from a low concentration to a high concentration. Kinetic profiles of antibody binding to 2019-nCoV RBD are shown in FIG. 3a (CB6 antibody) and FIG. 3b (GH12 antibody). The kinetic constants for antibody binding to 2019-nCoV RBD are shown in Table 6. Binding kinetic constants were calculated using BIAevaluation software 8K (Biacore, inc.) software. It can be seen that the CB6 and GH12 antibodies are able to bind to the 2019-nCoV RBD with higher affinity.

**Table 6. Kinetic constants for antibody binding to 2019-nCoV RBD protein**

| | ka (1/Ms) | kd (1/s) | KD (M) |
|---|---|---|---|
| CB6 | 8.95E+05 | 7.29E-04 | 8.15E-10 |
| GH12 | 2.95E+06 | 1.73E-02 | 5.87E-09 |

### Example 6: Detection of antibody of the present invention that block binding of 2019-nCoV RBD to ACE2

The gene encoding hACE2 (amino acid sequence shown in SEQ ID NO: 21) was constructed into pEGFP-N1 vector (purchased from Addgene) by Xhol and BamHI and expressed by fusion with GFP to form pEGFP-hACE2 plasmid. The pEGFP-hACE2 plasmid was transfected into HEK293T cells and GFP expression was observed under fluorescence microscope for 24 h. HEK293T-hACE2 cells were collected in 2 × 10⁵ per reaction and incubated with 2019-nCoV RBD (200 ng/mL) for 30 min at room temperature. After centrifugation at 500 × g for 5 min, the supernatant was removed and washed twice with PBS. After incubation with anti-His/APC (anti-His/APC) for 30 min at room temperature and washing twice with PBS, the cell surface fluorescence was detected with BD FACSCanto.

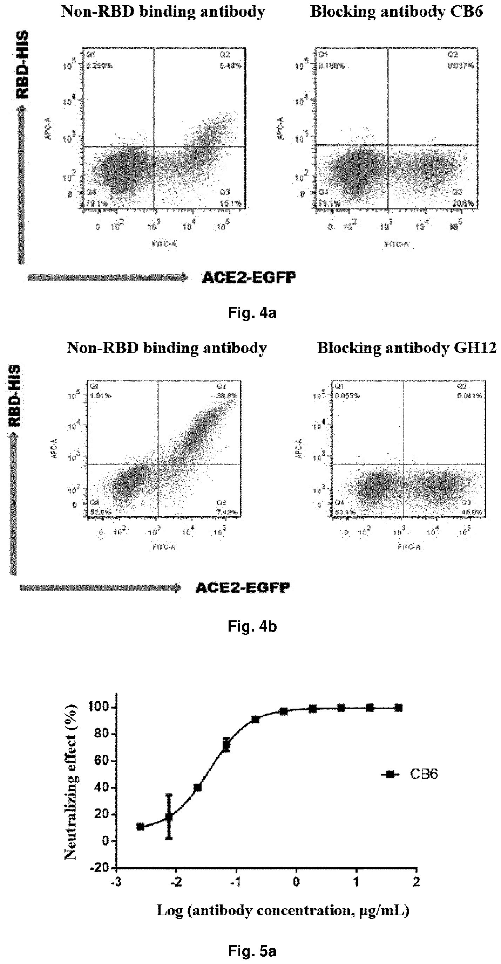

To examine the blocking effect of CB6 and GH12, the purified antibodies obtained in Example 4 (CB6 and GH12) were incubated with 200 ng/mL of 2019-nCoV RBD at a molar ratio of 10: 1 at room temperature for 1 h, respectively, and then incubated with HEK293T-hACE2 cells. The remaining steps are the same as above, using anti-His/APC to detect binding of protein to cells. The antibody blocks binding of 2019-nCoV RBD to HEK293T-hACE2 cells as shown in FIG. 4a (CB6 antibody) and FIG. 4b (GH12 antibody). Thus, both CB6 and GH12 antibodies blocked the binding of 2019-nCoV RBD to HEK293T-hACE2 cells.

### Example 7: Detection of antibody in the present invention neutralizing 2019-nCoV pseudovirus infection

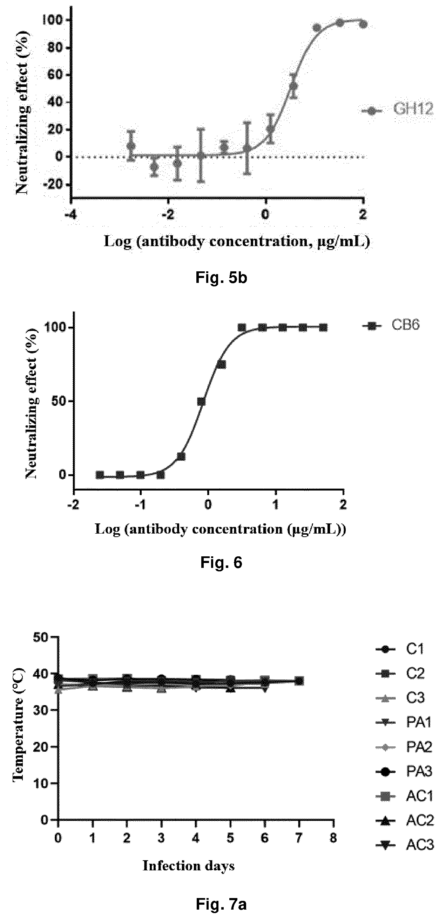

Purified antibodies (CB6 and GH12) obtained in Example 4 were diluted 3-fold starting from 50 µg/mL to a 10^{th} gradient (2.5 ng/mL), respectively, mixed with 1.6 × 10⁴ TCID₅₀ VSV-2019-nCoV pseudovirus, incubated for 1 h at 37°C, and then added to 96-well plates pre-inoculated with Huh7 cells (purchased from Basic Medical Cell Center of Peking Union Medical College). After incubation for 4 hours, the culture medium and virus solution were discarded, DMEM medium containing 10% FBS was added, and incubation was continued for 48 hours. The culture solution was discarded, washed once with PBS, added 1 × lysis solution (Promega, Luciferase Assay System) to lyse the cells, 10 µL of lysis solution was taken, 50 µL reaction substrate was added, and Promega Luminometers detection was performed. Neutralization ability of the antibody against VSV-2019-nCoV pseudovirus was calculated based on luciferase activity at different concentrations, and the results are shown in FIG. 5a (CB6 antibody) and FIG. 5b (GH12 antibody), and the results are statistically shown in Table 7.

**Table 7 Neutralizing effect of antibody on virus from different sources**

| mAbs | ND₅₀ (µg/mL)^{a} |
|---|---|
| CB6 | 0.00027 |
| GH12 | 3.27 |

| | |
|---|---|
| ^{a} Half inhibitory concentration | |

It can be seen that CB6 and GH12 antibodies can neutralize the 2019-nCoV pseudovirus with high neutralizing activity.

Taken together, CB6 and GH12 antibodies can serve as human monoclonal antibody with high neutralizing activity against a novel coronavirus (2019-nCoV).

### Example 8: Detection of antibody in the present invention neutralizing 2019-nCoV live virus

This study evaluated the neutralizing effect of CB6 antibody on the 2019-nCoV (SARS-CoV-2) live virus by in vitro neutralization assay.

### 8.1 Reagents

| **Name** | **Source** |
|---|---|
| COVID-19 SARS-CoV-2: BetaCoV/Wuhan/IVDC-HB-envF13/2020 | National Institute for Viral Disease Control and Prevention |
| Vero E6 cells | National Institute for Viral Disease Control and Prevention |
| FBS | Gibco |
| DMEM | Gibco |

### 8.2 Experimental method

Vero E6 cells were inoculated at a density of 1 × 10⁵ per well in 96-well culture plates and used after 24 hours at 37°C. In a 96-well tissue culture plate in DMEM medium, 50 µl of a serial 2-fold dilution of CB6 antibody (from 48.8 ng/mL to 100 µg/mL) was added. An equal volume of SARS-CoV-2 working stock containing 200 TCID₅₀ SARS-CoV-2 was then added for a final viral load of 100 TCID₅₀. The antibody-virus mixture was incubated for 1 h at 37°C and then transferred to a 96-well microtiter plate containing octameric fused Vero E6 cells and incubated for 3 days at 37°C in a CO₂ incubator. SARS-CoV-2 infected cells at 100 TCID₅₀ or cells cultured in control medium (DMEM + 10% FBS) were used as positive control or negative uninfected control, respectively. Cytopathic effect (CPE) was observed and recorded in each well before and after infection. Virus back titration was performed to evaluate the correct virus titration used in the experiment. The 50% neutralizing dose (NO₅₀) was calculated using Prism software. All experiments were performed in an approved biosafety level 3 facility in accordance with standard operating procedures.

### 8.3 Results and conclusions

The neutralizing function of CB6 was assessed by co-culturing cells with SARS-CoV-2 live virus in the presence of different concentrations of CB6. As shown in FIG. 6, CB6 reduced the cytopathic effect of SARS-CoV-2 virus on Vero E6 cells in a dose-dependent manner. The median neutralizing dose (ND₅₀) was 5.56 nM.

CB6 neutralizes SARS-CoV-2 live virus and reduces the pathological damage of the virus to cells.

### Example 9: Evaluation of the protective effect of the antibodies of the invention on animals

### 9.1 Experimental design

1) Non-human primate experimental animals are approximately 7-year-old rhesus monkeys (Hubei Tianqin Biotechnology Co. Ltd.), 3 females and 6 males, with body weight of 5.7-10.9 kg and age of 2512-2545 days. Routine pathogen detection and coronavirus viroid detection are completed according to the requirements of experimental animal quality management; the experimental animals meeting the requirements shall be adaptively reared in the laboratory for 3 days;
2) The control group, COVID-19 antibody treatment group and prevention group were set up, including three rhesus monkeys (C1, C2 and C3) in the control group, three rhesus monkeys (PA1, PA2 and PA3) in the prevention group and three rhesus monkeys (AC1, AC2 and AC3) in the treatment group. Wherein, the control group received a single injection of PBS 24 h before challenge, while the prevention group received CB6 at a dose of 50 mg/kg 24 h before challenge; the treatment group was injected with CB6 at 24 and 72 h after challenge at a dose of 50 mg/kg.
3) The animals were infected by endotracheal intubation, and 1 ml of virus solution was inoculated, and the virus inoculation amount was 1 × 10⁵ TCID₅₀;
4) Parameter collection:
   a. The change of disease course and health score of experimental animals were observed and recorded every day;
   b. After animals were anesthetized at different times, body weight and body temperature were measured, and anal swab, throat swab, alveolar lavage fluid and blood samples were collected;
   c. On day 3 and day 6 post-infection, the chest X-ray images of experimental animals were collected, and the time was synchronized with the observation and sampling;
   d. On day 5, day 6 and day 7 post-infection, one animal was randomly selected for necropsy, and different tissue and organ samples were collected. Histopathology, viral load and immunohistochemical analysis were performed on the samples after inactivation by conventional methods in the laboratory.
5) Virus load analysis, blood routine analysis, blood biochemical analysis and neutralizing antibody titer analysis of different samples (nasal swab, throat swab, anal swab, blood, tissue and organ) were completed, imaging of infected animals and pathological analysis of major organ tissues were completed;
6) According to the laboratory standard operating procedures and the requirements for biosafety management, all the collected animal tissue and organ samples shall be subjected to histopathological, viral load and immunohistochemical analysis after inactivation treatment in the laboratory; all waste generated during the experiment was disposed according to the waste management regulations of the laboratory.

### 9.2 Experimental method

### 1) Novel coronavirus strains

Novel coronavirus strain 2019-nCoV-WIV04 (GISAID accession number: EPI_ISI_402124) was isolated by Wuhan Institute of Virology, Chinese Academy of Sciences from a sample of bronchoalveolar lavage fluid from a patient with viral pneumonia in Wuhan in Dec. 2019. After the isolated virus strains are purified, cultured, proliferated and concentrated, 50% tissue cell infective dose (TCID₅₀) of the virus is determined, and the unit of virus titer is TCID₅₀/ml.

### 2) Animal behavior observation

During the immunization and virus challenge phase of experimental animals, the skin and hair, secretions, respiration, feces and urine, feed intake and exercise behavior, body weight and temperature were observed daily or at marked time, and the health of each animal was scored according to the "Clinical scoring criteria for rhesus monkeys after novel coronavirus challenge".

### 3) Virus titer determination

Novel coronavirus samples were inoculated into Vero E6 cells containing DMEM, and after 3 days of culture at 37°C and 5% CO₂, the culture solution was collected and stored in DMEM for future use. After the isolated virus strains are purified, cultured, proliferated and concentrated, 50% tissue cell infective dose (TCID₅₀) of the virus is determined. Vero E6 cells were titrated for virus by end-point titration. Ten-fold dilutions of the virus dilutions were inoculated to the cells and after 1 h incubation, the virus dilutions were aspirated and 100 µl DMEM (supplemented with 2% fetal bovine serum, 1 mm L-glutamine, penicillin (100 IU/ml) and streptomycin (100 µg/ml)) was added. Cytopathic score was performed after 3 days of culture and virus titer (TCID₅₀/ml) was calculated.

### 4) Real-time quantitative fluorescence PCR (qRT-PCR)

One-step real-time quantitative RT-PCR was used for quantitative analysis of viral RNA in the sample. Viral RNA was extracted from the swabs and blood samples using the QIAamp Viral RNA Mini Kit (Qiagen) according to the supplier's instructions. Samples were homogenized in DMEM (1:10, W/V), centrifuged at low speed at 4500 g for 30 min at 4°C, and RNA was immediately extracted from the supernatant. RNA was eluted in 50 µl of eluate as RT-PCR template. Based on the results of previous studies, primers for the S gene: RBD-qF1: 5'-CAATGGTTAAGGCAGG-3'; RBD-qR1: 5'-CTCAAGGTCTGGATCACG-3' were used.

The copy number of RNA in the samples was determined using the HiScript^{®} II One Step qRT-PCR SYBR^{®} Green Kit (Vazyme Biotech Co., Ltd) kit according to the instruction. 40 cycles of 50°C, 3 min, 95°C, 30 s, including termination at 95°C, 10 s, 60°C, 30 s were performed on an ABI 7700 machine and converted to virus copy number (Copies/ml) according to a standard curve.

### 5) Neutralizing antibody assay

Neutralizing antibody titers in serum samples were determined by neutralizing serum antibodies with a novel coronavirus live virus. The obtained animal serum samples were heat inactivated at 56°C for 30 min, diluted to 1:50, 1:150, 1:450, 1:1350, 1:4050 and 1:12150, added with equal amount of live virus, and incubated in an incubator containing 5% CO₂ at 37°C. After 1 h incubation, 100 microliters of the mixture were inoculated onto a monolayer of Vero cells in a 12-well plate and incubated for 1 h with shaking every 15 min. After removing the remaining inoculum, a culture medium of DMEM containing 0.9% methylcellulose and 2% FBS was added and incubated at 37°C, 5% CO₂ for 3 days. After 3 days, the cells were fixed with 4% formaldehyde for 30 min, the fixing solution was removed, rinsed with tap water, and then stained with crystal violet. The number of plaques was counted and the neutralizing antibody titer (EC₅₀) was determined.

### 6) Laboratory animal procedures

All challenge experiments were performed in a Level IV biosafety laboratory. Rhesus monkeys were anesthetized and inoculated with 1 ml of virus solution by endotracheal intubation. Animals will be observed and recorded daily for clinical symptoms including the nature, incidence, severity and duration of any severe or visible changes. Pulmonary X-ray was performed with HF100Ha (MIKASA, Japan) at different times to obtain pulmonary images and swab samples and blood samples from oropharynx, turbinate and anal region. The collected swab samples were placed in 1 ml of Dulbecco's modified Eagle's medium (DMEM) containing penicillin (100 IU/ml) and streptomycin (100 µg/ml). Whole blood was stored in K2 EDTA tubes for viral RNA extraction and blood routine analysis. To study the pathogenesis and pathological damage of respiratory tract, one animal was randomly euthanized on day 5, 6 and 7 after challenge. The trachea, right bronchus, left bronchus, six lung lobes and other tissues and organs were collected for pathological, virological and immunological analysis.

### 7) Animal anatomy and pathological analysis

Animal anatomy was performed according to the standard procedures for experimental animals. The collected organs and tissue samples were fixed in 10% neutral formalin buffer and then removed from the laboratory for paraffin embedding and sectioning. The sections were observed under light microscope after staining with hematoxylin and eosin. To detect the distribution of the novel coronavirus, paraffin-dehydrated tissue sections were placed in antigen buffer, blocked with 5% bovine serum albumin for 1 h at room temperature, and then blocked with a self-made primary antibody (rabbit anti-RP3-RP3-CoV N protein polyclonal antibody) at 1:500. After washing with PBS, sections were dried slightly, diluted at 1:200, and overlaid with Cy3-conjugated goat anti-rabbit IgG (Abcam) secondary antibody. Slides were washed with PBS and stained with 1:100 diluted DAPI. Images were acquired by the Pannoramic MIDI system (3DHISTECH, Budapest, Hungary).

According to the pathological changes in the lungs of experimental animals, determining the damage level of organs and tissues, wherein: "+++" indicates severe lesion; "++" is moderate lesion; "+" is mild lesion; "-" is no lesion and "+/-" is between mild and no lesion.

### 8) Other operations

According to the requirements for management system documents of biosafety level IV laboratory, use the standard operating procedures of laboratory to complete.

### 9.3 Experimental results

### 1) Selection and adaptive feeding of experimental animals

The experimental animals met the requirements for quality management of experimental animal in Hubei Province. The behavior, health and eating of the experimental animals were not abnormal after completing the three-day adaptive feeding in the laboratory. 2) Observation on clinical symptoms of experimental animals after virus inoculation The animals in control group, treatment group and prevention group were transferred to P4 laboratory. After 3-day adaptive feeding in the laboratory, the following operations were performed respectively.

Control and prevention groups: PBS and monoclonal antibody (up to 50 mg/kg) were intravenously injected indoors according to the plan. After 24 h, the animals were challenged by endotracheal intubation. Routine observation, body temperature and weight test were performed according to the experimental plan.

Treatment group: after 24 h challenge by endotracheal intubation, the drug was administered at day 1 and day 3 as planned, and routine observation, body temperature and body weight test were performed according to the experimental plan.

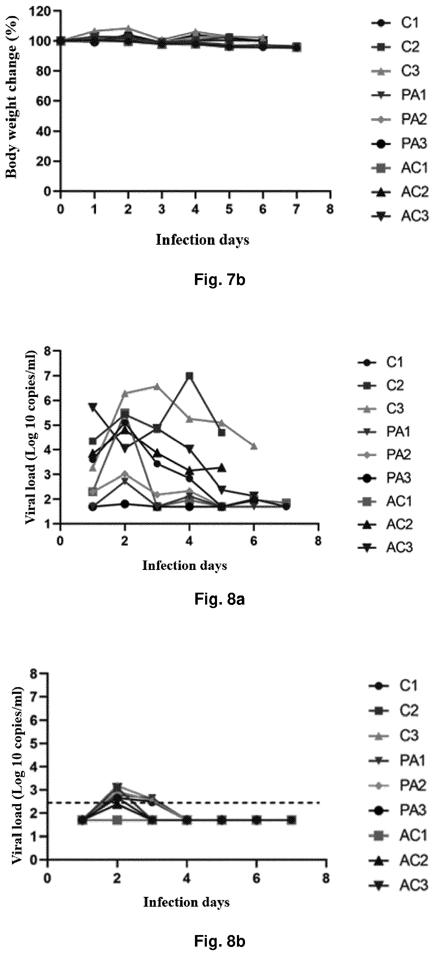

Results showed that the experimental animals of the control group, the COVID-19 antibody-treated group and the prevention group did not show significant behavioral differences throughout the experimental period, without significant changes in body temperature (see FIG. 7a) and body weight (see FIG. 7b).

### 3) Changes in viral load of throat swabs, nasal swabs and anal swabs

Throat swab, nasal swab and anal swab samples were collected daily to detect the change of viral load in different samples. Results showed that the virus load of throat swab in control group changed with the time of infection, and the virus load of experimental animals (C1, C2 and C3) reached the peak on the day 2 to day 4 after inoculation, and then decreased continuously. By day 7, the viral load in the samples dropped to a lower level. However, the virus load of throat swabs varied among different animals, and the peak of virus replication in C1, C2 and C3 was on the day 2, 3 and 4 after challenge, respectively. Overall, however, the variation of the viral load in the throat swab samples showed a proliferation process of the virus in vivo (see FIG. 8a). Compared with the detection results of viral load in the throat swab of control animals, throughout the experimental period, in PA1 and PA2 animals in the prevention group, low levels of viral nucleic acid could only be detected on day 2 and 3 after challenge, while the other detection points could not be detected. No viral nucleic acid was detected in PA3 animals throughout the experimental period.

There were significant individual differences in COVID-19 antibody treatment group; however, all reached the peak value of viral load at day 2 after challenge, and then continued to decrease, the viral load was far lower than that in the control group; the viral load of AC1 individuals at day 3 is lower than the lower limit of detection; Nasal swab samples, all groups had lower viral load; Low levels of viral nucleic acid copy number early in challenge were only detected in nasal swab samples and by day 7 no viral nucleic acid was detected in all nasal swab samples (see FIG. 8b).

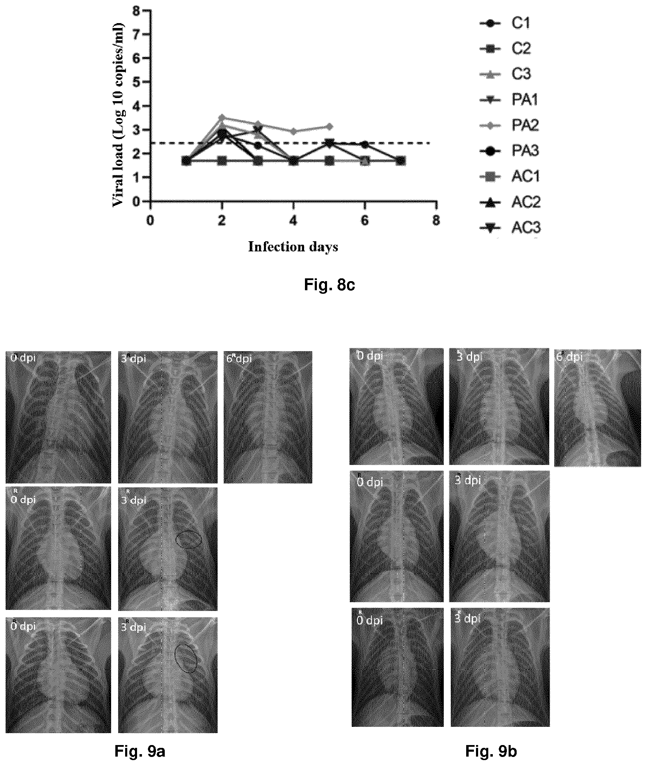

The viral load of anal swab samples was lower in all groups; Low levels of viral nucleic acid copy number early in challenge were only be detected in a few animals and by day 7 no viral nucleic acid was detected in all nasal swab samples (see FIG. 8c).

### 4) Imaging analysis of infected animals

X-ray lung images of 3 animals in the control group on day 0 of challenge were clear, and no pulmonary shadow was observed. On the day of virus challenge, no obvious shadow was observed in the lungs of experimental animals in the control group; On day 3 of challenge, one animal (C3) had a slightly blurred/disturbed lung texture, the other two animals (C1 and C2) had significant pulmonary shadow, and by day 6, no significant pulmonary shadow was seen (see FIG. 9a).

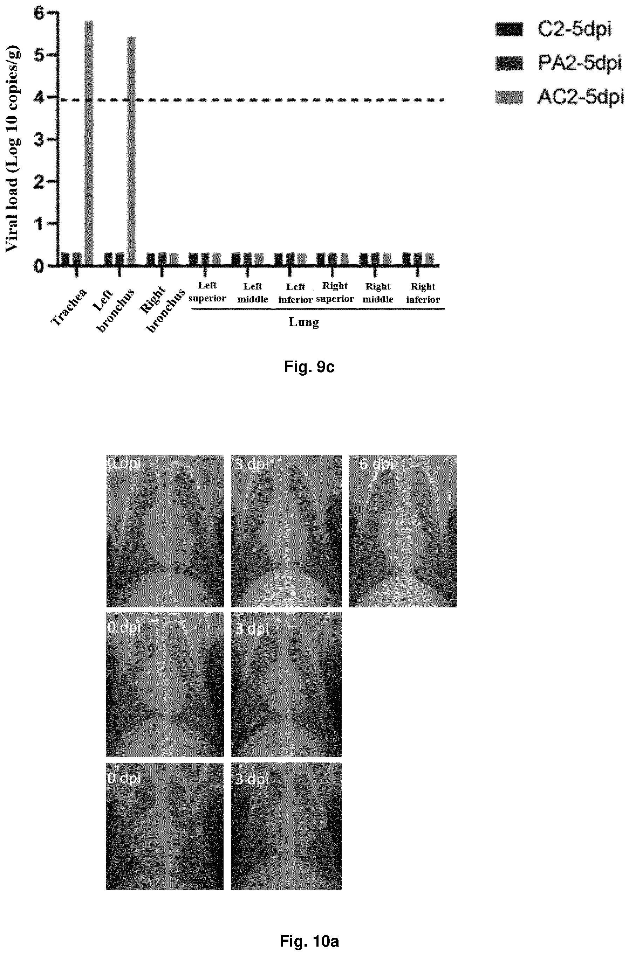

However, X-ray images showed no pulmonary shadow in all infected animals regardless of the 3 animals in the prevention group or the 3 animals in the treatment group on day 3 after challenge, slight blurred/disturbed pulmonary texture was observed, but no obvious pulmonary shadow was observed (see FIG. 9b and 9c).

### 5) Pathological change of infected animals

On day 5, 6 and 7 after infection, one animal in each of control group, prevention group and treatment group was randomly selected for dissection, and organs and tissues such as lung, trachea, bronchus, heart, liver, spleen, kidney, gastrointestinal tract, reproductive organs and lymph nodes were collected. According to the experimental requirements, the shape, color and tissue lesion site of each organ were observed.

On day 5 after challenge, animals in control group (C2) had light and off-white color on the general lung surface, moderate and slightly severe bilateral lower lung lesions, emphysema at the edge of lung lobe, and no obvious abnormality in trachea and bronchus. On day 6 and day 7 after challenge, animals in control group (D6: C3; D7: C1) had dark red general lung surface, moderate and slightly severe bilateral lower lung lesions, emphysema at the edge of lung lobe was mixed with lung consolidation area, and no obvious abnormality in trachea and bronchus. On day 5 after challenge, animals in the prevention group (PA2) and treatment group (AC2) had dark red general lung surface, moderate and slightly severe bilateral lower lung lesions, obvious lung lobe edge emphysema, and no obvious abnormality in trachea and bronchus. On day 6 and day 7 after challenge, experimental animals (prevention group: D6: PA3, D7: PA1; treatment group: D6: AC3, D7: AC1) had light and off-white color on the general lung surface, moderate and no obvious abnormality in lung, trachea and bronchus.

The histopathological changes of the lungs showed that on day 5 after infection, the changes of pulmonary diffuse interstitial pneumonia, alveolar wall thickening, fibroblast proliferation, fibrosis, and infiltration of monocytes and lymphocytes were observed in the control group; some alveolar edema and fibrin exudation, transparent membrane formation and pulmonary hemorrhage were observed. Transparent thrombosis was formed in part of pulmonary capillary lumen, and epithelial cells of bronchioles were necrotic and exfoliated. On day 6 and 7 after infection, pulmonary edema and fibrin exudation increased, alveolar macrophages increased, alveolar wall and alveolar fibrosis and organization were obvious. Compared with the control group, no matter the treatment group or the prevention group, the experimental animals had less lung injury caused by novel coronavirus. On day 5 after infection, the alveolar structures of two animals (AC2 and PA2) were basically intact, focal or patchy pulmonary fibrosis was observed, and mononuclear cells and lymphocyte infiltration were observed, but the number was significantly reduced compared with the control group, there were more macrophages in alveolar cavity, less edema, no transparent membrane formation, and no severe small bronchioles and pulmonary capillary lesions. On day 6 and 7 after infection, the pathological changes of animal lungs were significantly improved, the exudative lesions of lung tissue basically disappeared, and focal or patchy tissue fibrosis could be seen.

### 6) Viral load in different organ tissues

In order to further understand the distribution of virus in different tissues of upper respiratory tract and lung, the tissues of different parts of trachea, bronchi and lung of control group and two antibody group animals were collected on day 5, 6 and 7 after challenge, respectively, and the viral load in different organs and tissues was determined. The results showed that on day 5 after challenge, 4 - 8 × 10⁵ viral copies/g were detected in trachea and left bronchi of only one animal in prevention group (AC2), while no virosome was detected in other tissue samples, and no viral nucleic acid was detected in all treatment group animal samples (see FIG. 10a).

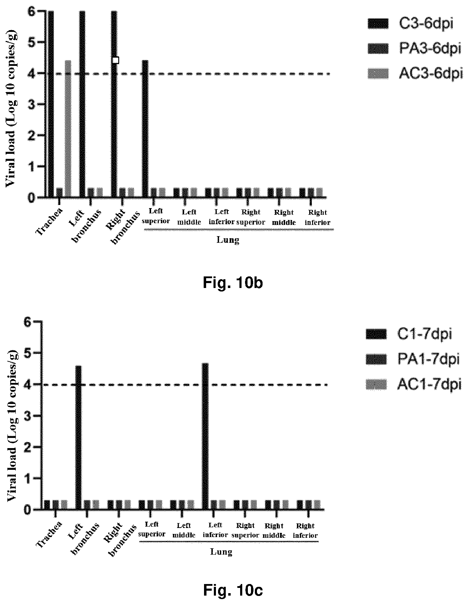

As shown in FIG. 10b, on day 6 after infection, 4 × 10⁵ - 1 × 10⁶ copies of virus/g were detected in the trachea, left and right bronchi, and upper lobe of left lung of control animals (C3), and no virosome was detected in other tissue samples. At the same time, 3 × 10⁵ viral copies/g were also detected in the tracheal tissue of one animal in the prevention group (AC3), and no virosome was detected in other tissue samples. No viral nucleic acid was detected in the treated animal samples.

As shown in FIG. 10c, on day 7 after infection, 5 × 10⁴ viral copies/g were detected in the left bronchus and lower lobe of the left lung of control animals (C1), and no virosome was detected in other tissue samples. At the same time, no virosome was detected in the tissue samples of animals in the prevention group and treatment group.

### 7) Immunohistochemical analysis

The results of immunohistochemical analysis of the virus in the lung tissue showed that the virus protein could be detected in the lungs from the control group on day 5, 6 and 7. However, the virus protein could not be detected in the lungs from the antibody treatment group and prevention group at different time.

The specific embodiments described above further illustrate the objects, technical solutions and advantages of the present invention. It should be understood that the foregoing description is only illustrative of specific embodiments of the invention, and is not intended to limit the invention. It is intended that the invention be construed as including all such modifications and alterations insofar as they come within the scope of the appended claims or the equivalents thereof.

## Claims

1. A human monoclonal antibody or antigen binding fragment thereof that specifically binds to a 2019-nCoV receptor binding domain (RBD), comprising a heavy chain variable region and/or a light chain variable region,
the heavy chain variable region comprising:
(I) HCDR1, HCDR2 and HCDR3 with the amino acid sequences shown in SEQ ID NO: 1, SEQ ID NO: 2 and SEQ ID NO: 3, respectively; or HCDR1, HCDR2 and HCDR3 having 1, 2 or 3 amino acid differences with the amino acid sequences shown in SEQ ID NO: 1, SEQ ID NO: 2 and SEQ ID NO: 3, respectively; or
(II) HCDR1, HCDR2 and HCDR3 with the amino acid sequences shown in SEQ ID NO: 25, SEQ ID NO: 26 and SEQ ID NO: 27, respectively; or HCDR1, HCDR2 and HCDR3 having 1, 2 or 3 amino acid differences with the amino acid sequences shown in SEQ ID NO: 25, SEQ ID NO: 26 and SEQ ID NO: 27, respectively;
the light chain variable region comprising:
(I) LCDR1, LCDR2 and LCDR3 with the amino acid sequences shown in SEQ ID NO: 4, SEQ ID NO: 5 and SEQ ID NO: 6, respectively; or LCDR1, LCDR2 and LCDR3 having 1, 2 or 3 amino acid differences with the amino acid sequences shown in SEQ ID NO: 4, SEQ ID NO: 5 and SEQ ID NO: 6, respectively; or
(II) LCDR1, LCDR2 and LCDR3 with the amino acid sequences shown in SEQ ID NO: 28, SEQ ID NO: 29 and SEQ ID NO: 30, respectively; or LCDR1, LCDR2 and LCDR3 having 1, 2 or 3 amino acid differences with the amino acid sequences shown in SEQ ID NO: 28, SEQ ID NO: 29 and SEQ ID NO: 30, respectively.

2. The human monoclonal antibody or antigen binding fragment thereof according to claim 1, wherein the antibody or antigen binding fragment thereof comprises a heavy chain variable region and/or a light chain variable region,
the heavy chain variable region comprising:
(I) HCDR1, HCDR2 and HCDR3 with the amino acid sequences shown in SEQ ID NO: 1, SEQ ID NO: 2 and SEQ ID NO: 3, respectively; or
(II) HCDR1, HCDR2 and HCDR3 with the amino acid sequences shown in SEQ ID NO: 25, SEQ ID NO: 26 and SEQ ID NO: 27, respectively;
the light chain variable region comprising:
(I) LCDR1, LCDR2 and LCDR3 with the amino acid sequences shown in SEQ ID NO: 4, SEQ ID NO: 5 and SEQ ID NO: 6, respectively; or
(II) LCDR1, LCDR2 and LCDR3 with the amino acid sequences shown in SEQ ID NO: 28, SEQ ID NO: 29 and SEQ ID NO: 30, respectively.

3. The human monoclonal antibody or antigen binding fragment thereof according to claim 2, wherein the antibody or antigen binding fragment thereof comprising:
(I) a heavy chain variable region having HCDR1, HCDR2 and HCDR3 with the amino acid sequences shown in SEQ ID NO: 1, SEQ ID NO: 2 and SEQ ID NO: 3, respectively; and a light chain variable region having LCDR1, LCDR2 and LCDR3 with the amino acid sequences shown in SEQ ID NO: 4, SEQ ID NO: 5 and SEQ ID NO: 6, respectively; or
(II) a heavy chain variable region having HCDR1, HCDR2 and HCDR3 with the amino acid sequences shown in SEQ ID NO: 25, SEQ ID NO: 26 and SEQ ID NO: 27, respectively; and a light chain variable region having LCDR1, LCDR2 and LCDR3 with the amino acid sequences shown in SEQ ID NO: 28, SEQ ID NO: 29 and SEQ ID NO: 30, respectively.

4. The human monoclonal antibody or antigen binding fragment thereof according to claim 3, comprising a heavy chain variable region and a light chain variable region:
(I) the heavy chain variable region comprising an amino acid sequence as shown in SEQ ID NO: 7, or an amino acid sequence having at least 95%, 96%, 97%, 98% or 99% sequence identity to the amino acid sequence as shown in SEQ ID NO: 7; and the light chain variable region comprising an amino acid sequence as shown in SEQ ID NO: 8, or an amino acid sequence having at least 95%, 96%, 97%, 98%, or 99% sequence identity to the amino acid sequence as shown in SEQ ID NO: 8; or
(II) the heavy chain variable region comprising an amino acid sequence as shown in SEQ ID NO: 31, or an amino acid sequence having at least 95%, 96%, 97%, 98% or 99% sequence identity to the amino acid sequence as shown in SEQ ID NO: 31; and the light chain variable region comprising an amino acid sequence as shown in SEQ ID NO: 32, or an amino acid sequence having at least 95%, 96%, 97%, 98% or 99% sequence identity to the amino acid sequence as shown in SEQ ID NO: 32.

5. The human monoclonal antibody or antigen binding fragment thereof according to claim 4, comprising:
(I) the heavy chain variable region having an amino acid sequence as shown in SEQ ID NO: 7 and the light chain variable region having an amino acid sequence as shown in SEQ ID NO: 8; or
(II) the heavy chain variable region having an amino acid sequence as shown in SEQ ID NO: 31 and the light chain variable region having an amino acid sequence as shown in SEQ ID NO: 32.

6. The human monoclonal antibody or antigen binding fragment thereof according to claim 5, wherein the antibody comprises a heavy chain and a light chain:
(I) the heavy chain comprises an amino acid sequence as shown in SEQ ID NO: 22, or an amino acid sequence having at least 90%, 92%, 94%, 95%, 96%, 97%, 98%, or 99% sequence identity to the amino acid sequence as shown in SEQ ID NO: 22; and the light chain comprises an amino acid sequence as shown in SEQ ID NO: 23, or an amino acid sequence having at least 90%, 92%, 94%, 95%, 96%, 97%, 98%, or 99% sequence identity to the amino acid sequence as shown in SEQ ID NO: 23; or
(II) the heavy chain comprises an amino acid sequence as shown in SEQ ID NO: 33, or an amino acid sequence having at least 90%, 92%, 94%, 95%, 96%, 97%, 98%, or 99% sequence identity to the amino acid sequence as shown in SEQ ID NO: 33; and the light chain comprises an amino acid sequence as shown in SEQ ID NO: 34, or an amino acid sequence having at least 90%, 92%, 94%, 95%, 96%, 97%, 98%, or 99% sequence identity to the amino acid sequence as shown in SEQ ID NO: 34.

7. The human monoclonal antibody or antigen binding fragment thereof according to claim 6, wherein the antibody comprises:
(I) a heavy chain having an amino acid sequence as shown in SEQ ID NO: 22, and a light chain having an amino acid sequence as shown in SEQ ID NO: 23; or
(II) a heavy chain having an amino acid sequence as shown in SEQ ID NO: 33, and a light chain having an amino acid sequence as shown in SEQ ID NO: 34.

8. The human monoclonal antibody or antigen binding fragment thereof according to any one of claims 1-7, wherein the antigen binding fragment is selected from Fab, Fab', Fab'-SH, Fv, scFv, F(ab')2, diabody.

9. A polypeptide comprising a sequence selected from SEQ ID NOs: 7, 8, 22 or 23.

10. A polypeptide comprising a sequence selected from SEQ ID NOs: 31, 32, 33 or 34, wherein the polypeptide is part of a human monoclonal antibody that specifically binds to a 2019-nCoV receptor binding domain (RBD), and
when the polypeptide comprises SEQ ID NO: 31, the human monoclonal antibody further comprises the polypeptide as shown in SEQ ID NO: 32;
when the polypeptide comprises SEQ ID NO: 32, the human monoclonal antibody further comprises the polypeptide as shown in SEQ ID NO: 31;
when the polypeptide comprises SEQ ID NO: 33, the human monoclonal antibody further comprises the polypeptide as shown in SEQ ID NO: 34; or
when the polypeptide comprises SEQ ID NO: 34, the human monoclonal antibody further comprises the polypeptide as shown in SEQ ID NO: 33.

11. A polynucleotide encoding the human monoclonal antibody or antigen binding fragment thereof according to any one of claims 1-8 or the polypeptide according to claim 9 or 10.

12. An expression vector comprising the polynucleotide according to claim 11, preferably the vector is a eukaryotic expression vector.

13. A host cell comprising the polynucleotide according to claim 11 or the expression vector according to claim 12, preferably the host cell is a eukaryotic cell, more preferably a mammalian cell.

14. A method for preparing the human monoclonal antibody or antigen binding fragment thereof according to any one of claims 1-8 or the polypeptide according to claim 9 or 10, the method comprising expressing the antibody or antigen binding fragment thereof or the polypeptide in the host cell according to claim 13 under conditions suitable for expression of the antibody or antigen binding fragment thereof or the polypeptide, and recovering the expressed antibody or antigen binding fragment thereof or the polypeptide from the host cell.

15. A pharmaceutical composition comprising the human monoclonal antibody or antigen binding fragment thereof according to any one of claims 1-8, the polypeptide according to claim 9 or 10, the polynucleotide according to claim 11, the expression vector according to claim 12 and/or the host cell according to claim 13, and a pharmaceutically acceptable carrier.

16. Use of the human monoclonal antibody or antigen binding fragment thereof according to any one of claims 1-8, the polypeptide according to claim 9 or 10, the polynucleotide according to claim 11, the expression vector according to claim 12, the host cell according to claim 13 and/or the pharmaceutical composition according to claim 15 for the preparation of a medicament for the treatment and/or prevention of a 2019-nCoV infection.

17. A kit comprising the human monoclonal antibody or antigen binding fragment thereof according to any one of claims 1-8, the polypeptide of claim 9 or 10, the polynucleotide according to claim 11, the expression vector according to claim 12, the host cell according to claim 13 and/or the pharmaceutical composition according to claim 15.

18. Use of the kit according to claim 17 for the preparation of a medicament for the diagnosis of a 2019-nCoV infection.

19. A method for detecting the presence of a 2019-nCoV in a sample, comprising using the human monoclonal antibody or antigen binding fragment thereof according to any one of claims 1-8 or the polypeptide according to claim 9 or 10.

20. A method for treating and/or preventing a 2019-nCoV infection, comprising administering to a subject in need thereof the human monoclonal antibody or antigen binding fragment thereof according to any one of claims 1-8, the polypeptide according to claim 9 or 10, the polynucleotide according to claim 11, the expression vector according to claim 12, the host cell according to claim 13, and/or the pharmaceutical composition according to claim 15.


SEQUENCE LISTING

<110>  INSTITUTE OF MICROBIOLOGY, CHINESE ACADEMY OF SCIENCES

<120>  HUMANIZED MONOCLONAL ANTIBODY FOR 2019 NOVEL CORONAVIRUS AND USE
THEREOF

<130>  MTPIUS20238

<140>  PCT/CN2021/077392
<141>  2021-02-23

<150>  CN202010114283.8
<151>  2020-02-24

<150>  CN202010137486.9
<151>  2020-03-02

<150>  CN202110044541.4
<151>  2021-01-13

<160>  34

<170>  SIPOSequenceListing 1.0

<210>  1
<211>  8
<212>  PRT
<213>  Artificial Sequence

<220>
<223>  synthetical protein

<400>  1
Gly Phe Thr Val Ser Ser Asn Tyr
1               5

<210>  2
<211>  7
<212>  PRT
<213>  Artificial Sequence

<220>
<223>  synthetical protein

<400>  2
Ile Tyr Ser Gly Gly Ser Thr
1               5

<210>  3
<211>  13
<212>  PRT
<213>  Artificial Sequence

<220>
<223>  synthetical protein

<400>  3
Ala Arg Val Leu Pro Met Tyr Gly Asp Tyr Leu Asp Tyr
1               5                   10

<210>  4
<211>  6

<212>  PRT
<213>  Artificial Sequence

<220>
<223>  synthetical protein

<400>  4
Gln Ser Ile Ser Arg Tyr
1               5

<210>  5
<211>  3
<212>  PRT
<213>  Artificial Sequence

<220>
<223>  synthetical protein

<400>  5
Ala Ala Ser
1

<210>  6
<211>  11
<212>  PRT
<213>  Artificial Sequence

<220>
<223>  synthetical protein

<400>  6
Gln Gln Ser Tyr Ser Thr Pro Pro Glu Tyr Thr
1               5                   10

<210>  7
<211>  119
<212>  PRT
<213>  Artificial Sequence

<220>
<223>  synthetical protein

<400>  7
Glu Val Gln Leu Val Glu Ser Gly Gly Gly Leu Val Gln Pro Gly Gly
1               5                   10                  15
Ser Leu Arg Leu Ser Cys Ala Ala Ser Gly Phe Thr Val Ser Ser Asn
            20                  25                  30
Tyr Met Ser Trp Val Arg Gln Ala Pro Gly Lys Gly Leu Glu Trp Val
        35                  40                  45
Ser Val Ile Tyr Ser Gly Gly Ser Thr Phe Tyr Ala Asp Ser Val Lys
    50                  55                  60
Gly Arg Phe Thr Ile Ser Arg Asp Asn Ser Met Asn Thr Leu Phe Leu
65                  70                  75                  80
Gln Met Asn Ser Leu Arg Ala Glu Asp Thr Ala Val Tyr Tyr Cys Ala
                85                  90                  95
Arg Val Leu Pro Met Tyr Gly Asp Tyr Leu Asp Tyr Trp Gly Gln Gly
            100                 105                 110
Thr Leu Val Thr Val Ser Ser
        115

<210>  8
<211>  109
<212>  PRT

<213>  Artificial Sequence

<220>
<223>  synthetical protein

<400>  8
Asp Ile Val Met Thr Gln Ser Pro Ser Ser Leu Ser Ala Ser Val Gly
1               5                   10                  15
Asp Arg Val Thr Ile Thr Cys Arg Ala Ser Gln Ser Ile Ser Arg Tyr
            20                  25                  30
Leu Asn Trp Tyr Gln Gln Lys Pro Gly Lys Ala Pro Lys Leu Leu Ile
        35                  40                  45
Tyr Ala Ala Ser Ser Leu Gln Ser Gly Val Pro Ser Arg Phe Ser Gly
    50                  55                  60
Ser Gly Ser Gly Thr Asp Phe Thr Leu Thr Ile Ser Ser Leu Gln Pro
65                  70                  75                  80
Glu Asp Phe Ala Thr Tyr Tyr Cys Gln Gln Ser Tyr Ser Thr Pro Pro
                85                  90                  95
Glu Tyr Thr Phe Gly Gln Gly Thr Lys Leu Glu Ile Lys
            100                 105

<210>  9
<211>  229
<212>  PRT
<213>  2019-nCoV

<400>  9
Arg Val Gln Pro Thr Glu Ser Ile Val Arg Phe Pro Asn Ile Thr Asn
1               5                   10                  15
Leu Cys Pro Phe Gly Glu Val Phe Asn Ala Thr Arg Phe Ala Ser Val
            20                  25                  30
Tyr Ala Trp Asn Arg Lys Arg Ile Ser Asn Cys Val Ala Asp Tyr Ser
        35                  40                  45
Val Leu Tyr Asn Ser Ala Ser Phe Ser Thr Phe Lys Cys Tyr Gly Val
    50                  55                  60
Ser Pro Thr Lys Leu Asn Asp Leu Cys Phe Thr Asn Val Tyr Ala Asp
65                  70                  75                  80
Ser Phe Val Ile Arg Gly Asp Glu Val Arg Gln Ile Ala Pro Gly Gln
                85                  90                  95
Thr Gly Lys Ile Ala Asp Tyr Asn Tyr Lys Leu Pro Asp Asp Phe Thr
            100                 105                 110
Gly Cys Val Ile Ala Trp Asn Ser Asn Asn Leu Asp Ser Lys Val Gly
        115                 120                 125
Gly Asn Tyr Asn Tyr Leu Tyr Arg Leu Phe Arg Lys Ser Asn Leu Lys
    130                 135                 140
Pro Phe Glu Arg Asp Ile Ser Thr Glu Ile Tyr Gln Ala Gly Ser Thr
145                 150                 155                 160
Pro Cys Asn Gly Val Glu Gly Phe Asn Cys Tyr Phe Pro Leu Gln Ser
                165                 170                 175
Tyr Gly Phe Gln Pro Thr Asn Gly Val Gly Tyr Gln Pro Tyr Arg Val
            180                 185                 190
Val Val Leu Ser Phe Glu Leu Leu His Ala Pro Ala Thr Val Cys Gly
        195                 200                 205
Pro Lys Lys Ser Thr Asn Leu Val Lys Asn Lys Cys Val Asn Phe His
    210                 215                 220
His His His His His
225

<210>  10
<211>  20
<212>  DNA
<213>  Artificial Sequence

<220>
<223>  synthetical nucleic acid

<400>  10
atggagtcgg gaaggaagtc                                                   20

<210>  11
<211>  19
<212>  DNA
<213>  Artificial Sequence

<220>
<223>  synthetical nucleic acid

<400>  11
tcacggacgt tgggtggta                                                    19

<210>  12
<211>  19
<212>  DNA
<213>  Artificial Sequence

<220>
<223>  synthetical nucleic acid

<400>  12
tcacggaggt ggcattgga                                                    19

<210>  13
<211>  19
<212>  DNA
<213>  Artificial Sequence

<220>
<223>  synthetical nucleic acid

<400>  13
caggcgatga ccacgttcc                                                    19

<210>  14
<211>  19
<212>  DNA
<213>  Artificial Sequence

<220>
<223>  synthetical nucleic acid

<400>  14
catgcgacga ccacgttcc                                                    19

<210>  15
<211>  20
<212>  DNA
<213>  Artificial Sequence

<220>
<223>  synthetical nucleic acid

<400>  15
aggtgtgcac gccgctggtc                                                   20

<210>  16
<211>  20

<212>  DNA
<213>  Artificial Sequence

<220>
<223>  synthetical nucleic acid

<400>  16
gcaggcacac aacagaggca                                                    20

<210>  17
<211>  17
<212>  DNA
<213>  Artificial Sequence

<220>
<223>  synthetical nucleic acid

<400>  17
aggccactgt cacagct                                                       17

<210>  18
<211>  21
<212>  PRT
<213>  Artificial Sequence

<220>
<223>  synthetical protein

<400>  18
Met Glu Thr Asp Thr Leu Leu Leu Trp Val Leu Leu Leu Trp Val Pro
1               5                   10                  15
Gly Ser Thr Gly Asp
            20

<210>  19
<211>  330
<212>  PRT
<213>  Artificial Sequence

<220>
<223>  synthetical protein

<400>  19
Ala Ser Thr Lys Gly Pro Ser Val Phe Pro Leu Ala Pro Ser Ser Lys
1               5                   10                  15
Ser Thr Ser Gly Gly Thr Ala Ala Leu Gly Cys Leu Val Lys Asp Tyr
            20                  25                  30
Phe Pro Glu Pro Val Thr Val Ser Trp Asn Ser Gly Ala Leu Thr Ser
        35                  40                  45
Gly Val His Thr Phe Pro Ala Val Leu Gln Ser Ser Gly Leu Tyr Ser
    50                  55                  60
Leu Ser Ser Val Val Thr Val Pro Ser Ser Ser Leu Gly Thr Gln Thr
65                  70                  75                  80
Tyr Ile Cys Asn Val Asn His Lys Pro Ser Asn Thr Lys Val Asp Lys
                85                  90                  95
Arg Val Glu Pro Lys Ser Cys Asp Lys Thr His Thr Cys Pro Pro Cys
            100                 105                 110
Pro Ala Pro Glu Leu Leu Gly Gly Pro Ser Val Phe Leu Phe Pro Pro
        115                 120                 125
Lys Pro Lys Asp Thr Leu Met Ile Ser Arg Thr Pro Glu Val Thr Cys
    130                 135                 140
Val Val Val Asp Val Ser His Glu Asp Pro Glu Val Lys Phe Asn Trp
145                 150                 155                 160

Tyr Val Asp Gly Val Glu Val His Asn Ala Lys Thr Lys Pro Arg Glu
                165                 170                 175
Glu Gln Tyr Asn Ser Thr Tyr Arg Val Val Ser Val Leu Thr Val Leu
            180                 185                 190
His Gln Asp Trp Leu Asn Gly Lys Glu Tyr Lys Cys Lys Val Ser Asn
        195                 200                 205
Lys Ala Leu Pro Ala Pro Ile Glu Lys Thr Ile Ser Lys Ala Lys Gly
    210                 215                 220
Gln Pro Arg Glu Pro Gln Val Tyr Thr Leu Pro Pro Ser Arg Asp Glu
225                 230                 235                 240
Leu Thr Lys Asn Gln Val Ser Leu Thr Cys Leu Val Lys Gly Phe Tyr
                245                 250                 255
Pro Ser Asp Ile Ala Val Glu Trp Glu Ser Asn Gly Gln Pro Glu Asn
            260                 265                 270
Asn Tyr Lys Thr Thr Pro Pro Val Leu Asp Ser Asp Gly Ser Phe Phe
        275                 280                 285
Leu Tyr Ser Lys Leu Thr Val Asp Lys Ser Arg Trp Gln Gln Gly Asn
    290                 295                 300
Val Phe Ser Cys Ser Val Met His Glu Ala Leu His Asn His Tyr Thr
305                 310                 315                 320
Gln Lys Ser Leu Ser Leu Ser Pro Gly Lys
                325                 330

<210>  20
<211>  105
<212>  PRT
<213>  Artificial Sequence

<220>
<223>  synthetical protein

<400>  20
Ala Ala Pro Ser Val Phe Ile Phe Pro Pro Ser Asp Glu Gln Leu Lys
1               5                   10                  15
Ser Gly Thr Ala Ser Val Val Cys Leu Leu Asn Asn Phe Tyr Pro Arg
            20                  25                  30
Glu Ala Lys Val Gln Trp Lys Val Asp Asn Ala Leu Gln Ser Gly Asn
        35                  40                  45
Ser Gln Glu Ser Val Thr Glu Gln Asp Ser Lys Asp Ser Thr Tyr Ser
    50                  55                  60
Leu Ser Ser Thr Leu Thr Leu Ser Lys Ala Asp Tyr Glu Lys His Lys
65                  70                  75                  80
Val Tyr Ala Cys Glu Val Thr His Gln Gly Leu Ser Ser Pro Val Thr
                85                  90                  95
Lys Ser Phe Asn Arg Gly Glu Cys Ser
            100                 105

<210>  21
<211>  805
<212>  PRT
<213>  Homo Sapiens

<400>  21
Met Ser Gly Ser Phe Trp Leu Leu Leu Ser Leu Val Ala Val Thr Ala
1               5                   10                  15
Ala Gln Ser Thr Ile Glu Glu Gln Ala Lys Thr Phe Leu Asp Lys Phe
            20                  25                  30
Asn His Glu Ala Glu Asp Leu Phe Tyr Gln Ser Ser Leu Ala Ser Trp
        35                  40                  45
Asn Tyr Asn Thr Asn Ile Thr Glu Glu Asn Val Gln Asn Met Asn Asn
    50                  55                  60
Ala Gly Asp Lys Trp Ser Ala Phe Leu Lys Glu Gln Ser Thr Leu Ala
65                  70                  75                  80

Gln Met Tyr Pro Leu Gln Glu Ile Gln Asn Leu Thr Val Lys Leu Gln
                85                  90                  95
Leu Gln Ala Leu Gln Gln Asn Gly Ser Ser Val Leu Ser Glu Asp Lys
            100                 105                 110
Ser Lys Arg Leu Asn Thr Ile Leu Asn Thr Met Ser Thr Ile Tyr Ser
        115                 120                 125
Thr Gly Lys Val Cys Asn Pro Asp Asn Pro Gln Glu Cys Leu Leu Leu
    130                 135                 140
Glu Pro Gly Leu Asn Glu Ile Met Ala Asn Ser Leu Asp Tyr Asn Glu
145                 150                 155                 160
Arg Leu Trp Ala Trp Glu Ser Trp Arg Ser Glu Val Gly Lys Gln Leu
                165                 170                 175
Arg Pro Leu Tyr Glu Glu Tyr Val Val Leu Lys Asn Glu Met Ala Arg
            180                 185                 190
Ala Asn His Tyr Glu Asp Tyr Gly Asp Tyr Trp Arg Gly Asp Tyr Glu
        195                 200                 205
Val Asn Gly Val Asp Gly Tyr Asp Tyr Ser Arg Gly Gln Leu Ile Glu
    210                 215                 220
Asp Val Glu His Thr Phe Glu Glu Ile Lys Pro Leu Tyr Glu His Leu
225                 230                 235                 240
His Ala Tyr Val Arg Ala Lys Leu Met Asn Ala Tyr Pro Ser Tyr Ile
                245                 250                 255
Ser Pro Ile Gly Cys Leu Pro Ala His Leu Leu Gly Asp Met Trp Gly
            260                 265                 270
Arg Phe Trp Thr Asn Leu Tyr Ser Leu Thr Val Pro Phe Gly Gln Lys
        275                 280                 285
Pro Asn Ile Asp Val Thr Asp Ala Met Val Asp Gln Ala Trp Asp Ala
    290                 295                 300
Gln Arg Ile Phe Lys Glu Ala Glu Lys Phe Phe Val Ser Val Gly Leu
305                 310                 315                 320
Pro Asn Met Thr Gln Gly Phe Trp Glu Asn Ser Met Leu Thr Asp Pro
                325                 330                 335
Gly Asn Val Gln Lys Ala Val Cys His Pro Thr Ala Trp Asp Leu Gly
            340                 345                 350
Lys Gly Asp Phe Arg Ile Leu Met Cys Thr Lys Val Thr Met Asp Asp
        355                 360                 365
Phe Leu Thr Ala His His Glu Met Gly His Ile Gln Tyr Asp Met Ala
    370                 375                 380
Tyr Ala Ala Gln Pro Phe Leu Leu Arg Asn Gly Ala Asn Glu Gly Phe
385                 390                 395                 400
His Glu Ala Val Gly Glu Ile Met Ser Leu Ser Ala Ala Thr Pro Lys
                405                 410                 415
His Leu Lys Ser Ile Gly Leu Leu Ser Pro Asp Phe Gln Glu Asp Asn
            420                 425                 430
Glu Thr Glu Ile Asn Phe Leu Leu Lys Gln Ala Leu Thr Ile Val Gly
        435                 440                 445
Thr Leu Pro Phe Thr Tyr Met Leu Glu Lys Trp Arg Trp Met Val Phe
    450                 455                 460
Lys Gly Glu Ile Pro Lys Asp Gln Trp Met Lys Lys Trp Trp Glu Met
465                 470                 475                 480
Lys Arg Glu Ile Val Gly Val Val Glu Pro Val Pro His Asp Glu Thr
                485                 490                 495
Tyr Cys Asp Pro Ala Ser Leu Phe His Val Ser Asn Asp Tyr Ser Phe
            500                 505                 510
Ile Arg Tyr Tyr Thr Arg Thr Leu Tyr Gln Phe Gln Phe Gln Glu Ala
        515                 520                 525
Leu Cys Gln Ala Ala Lys His Glu Gly Pro Leu His Lys Cys Asp Ile
    530                 535                 540
Ser Asn Ser Thr Glu Ala Gly Gln Lys Leu Phe Asn Met Leu Arg Leu
545                 550                 555                 560
Gly Lys Ser Glu Pro Trp Thr Leu Ala Leu Glu Asn Val Val Gly Ala
                565                 570                 575
Lys Asn Met Asn Val Arg Pro Leu Leu Asn Tyr Phe Glu Pro Leu Phe

580                 585                 590
Thr Trp Leu Lys Asp Gln Asn Lys Asn Ser Phe Val Gly Trp Ser Thr
        595                 600                 605
Asp Trp Ser Pro Tyr Ala Asp Gln Ser Ile Lys Val Arg Ile Ser Leu
    610                 615                 620
Lys Ser Ala Leu Gly Asp Lys Ala Tyr Glu Trp Asn Asp Asn Glu Met
625                 630                 635                 640
Tyr Leu Phe Arg Ser Ser Val Ala Tyr Ala Met Arg Gln Tyr Phe Leu
                645                 650                 655
Lys Val Lys Asn Gln Met Ile Leu Phe Gly Glu Glu Asp Val Arg Val
            660                 665                 670
Ala Asn Leu Lys Pro Arg Ile Ser Phe Asn Phe Phe Val Thr Ala Pro
        675                 680                 685
Lys Asn Val Ser Asp Ile Ile Pro Arg Thr Glu Val Glu Lys Ala Ile
    690                 695                 700
Arg Met Ser Arg Ser Arg Ile Asn Asp Ala Phe Arg Leu Asn Asp Asn
705                 710                 715                 720
Ser Leu Glu Phe Leu Gly Ile Gln Pro Thr Leu Gly Pro Pro Asn Gln
                725                 730                 735
Pro Pro Val Ser Ile Trp Leu Ile Val Phe Gly Val Val Met Gly Val
            740                 745                 750
Ile Val Val Gly Ile Val Ile Leu Ile Phe Thr Gly Ile Arg Asp Arg
        755                 760                 765
Lys Lys Lys Asn Lys Ala Arg Ser Gly Glu Asn Pro Tyr Ala Ser Ile
    770                 775                 780
Asp Ile Ser Lys Gly Glu Asn Asn Pro Gly Phe Gln Asn Thr Asp Asp
785                 790                 795                 800
Val Gln Thr Ser Phe
                805

<210>  22
<211>  449
<212>  PRT
<213>  Artificial Sequence

<220>
<223>  synthetical protein

<400>  22
Glu Val Gln Leu Val Glu Ser Gly Gly Gly Leu Val Gln Pro Gly Gly
1               5                   10                  15
Ser Leu Arg Leu Ser Cys Ala Ala Ser Gly Phe Thr Val Ser Ser Asn
            20                  25                  30
Tyr Met Ser Trp Val Arg Gln Ala Pro Gly Lys Gly Leu Glu Trp Val
        35                  40                  45
Ser Val Ile Tyr Ser Gly Gly Ser Thr Phe Tyr Ala Asp Ser Val Lys
    50                  55                  60
Gly Arg Phe Thr Ile Ser Arg Asp Asn Ser Met Asn Thr Leu Phe Leu
65                  70                  75                  80
Gln Met Asn Ser Leu Arg Ala Glu Asp Thr Ala Val Tyr Tyr Cys Ala
                85                  90                  95
Arg Val Leu Pro Met Tyr Gly Asp Tyr Leu Asp Tyr Trp Gly Gln Gly
            100                 105                 110
Thr Leu Val Thr Val Ser Ser Ala Ser Thr Lys Gly Pro Ser Val Phe
        115                 120                 125
Pro Leu Ala Pro Ser Ser Lys Ser Thr Ser Gly Gly Thr Ala Ala Leu
    130                 135                 140
Gly Cys Leu Val Lys Asp Tyr Phe Pro Glu Pro Val Thr Val Ser Trp
145                 150                 155                 160
Asn Ser Gly Ala Leu Thr Ser Gly Val His Thr Phe Pro Ala Val Leu
                165                 170                 175
Gln Ser Ser Gly Leu Tyr Ser Leu Ser Ser Val Val Thr Val Pro Ser
            180                 185                 190

Ser Ser Leu Gly Thr Gln Thr Tyr Ile Cys Asn Val Asn His Lys Pro
        195                 200                 205
Ser Asn Thr Lys Val Asp Lys Arg Val Glu Pro Lys Ser Cys Asp Lys
    210                 215                 220
Thr His Thr Cys Pro Pro Cys Pro Ala Pro Glu Leu Leu Gly Gly Pro
225                 230                 235                 240
Ser Val Phe Leu Phe Pro Pro Lys Pro Lys Asp Thr Leu Met Ile Ser
                245                 250                 255
Arg Thr Pro Glu Val Thr Cys Val Val Val Asp Val Ser His Glu Asp
            260                 265                 270
Pro Glu Val Lys Phe Asn Trp Tyr Val Asp Gly Val Glu Val His Asn
        275                 280                 285
Ala Lys Thr Lys Pro Arg Glu Glu Gln Tyr Asn Ser Thr Tyr Arg Val
    290                 295                 300
Val Ser Val Leu Thr Val Leu His Gln Asp Trp Leu Asn Gly Lys Glu
305                 310                 315                 320
Tyr Lys Cys Lys Val Ser Asn Lys Ala Leu Pro Ala Pro Ile Glu Lys
                325                 330                 335
Thr Ile Ser Lys Ala Lys Gly Gln Pro Arg Glu Pro Gln Val Tyr Thr
            340                 345                 350
Leu Pro Pro Ser Arg Asp Glu Leu Thr Lys Asn Gln Val Ser Leu Thr
        355                 360                 365
Cys Leu Val Lys Gly Phe Tyr Pro Ser Asp Ile Ala Val Glu Trp Glu
    370                 375                 380
Ser Asn Gly Gln Pro Glu Asn Asn Tyr Lys Thr Thr Pro Pro Val Leu
385                 390                 395                 400
Asp Ser Asp Gly Ser Phe Phe Leu Tyr Ser Lys Leu Thr Val Asp Lys
                405                 410                 415
Ser Arg Trp Gln Gln Gly Asn Val Phe Ser Cys Ser Val Met His Glu
            420                 425                 430
Ala Leu His Asn His Tyr Thr Gln Lys Ser Leu Ser Leu Ser Pro Gly
        435                 440                 445
Lys

<210>  23
<211>  217
<212>  PRT
<213>  Artificial Sequence

<220>
<223>  synthetical protein

<400>  23
Asp Ile Val Met Thr Gln Ser Pro Ser Ser Leu Ser Ala Ser Val Gly
1               5                   10                  15
Asp Arg Val Thr Ile Thr Cys Arg Ala Ser Gln Ser Ile Ser Arg Tyr
            20                  25                  30
Leu Asn Trp Tyr Gln Gln Lys Pro Gly Lys Ala Pro Lys Leu Leu Ile
        35                  40                  45
Tyr Ala Ala Ser Ser Leu Gln Ser Gly Val Pro Ser Arg Phe Ser Gly
    50                  55                  60
Ser Gly Ser Gly Thr Asp Phe Thr Leu Thr Ile Ser Ser Leu Gln Pro
65                  70                  75                  80
Glu Asp Phe Ala Thr Tyr Tyr Cys Gln Gln Ser Tyr Ser Thr Pro Pro
                85                  90                  95
Glu Tyr Thr Phe Gly Gln Gly Thr Lys Leu Glu Ile Lys Arg Thr Val
            100                 105                 110
Ala Ala Pro Ser Val Phe Ile Phe Pro Pro Ser Asp Glu Gln Leu Lys
        115                 120                 125
Ser Gly Thr Ala Ser Val Val Cys Leu Leu Asn Asn Phe Tyr Pro Arg
    130                 135                 140
Glu Ala Lys Val Gln Trp Lys Val Asp Asn Ala Leu Gln Ser Gly Asn

145                 150                 155                 160
Ser Gln Glu Ser Val Thr Glu Gln Asp Ser Lys Asp Ser Thr Tyr Ser
                165                 170                 175
Leu Ser Ser Thr Leu Thr Leu Ser Lys Ala Asp Tyr Glu Lys His Lys
            180                 185                 190
Val Tyr Ala Cys Glu Val Thr His Gln Gly Leu Ser Ser Pro Val Thr
        195                 200                 205
Lys Ser Phe Asn Arg Gly Glu Cys Ser
    210                 215

<210>  24
<211>  102
<212>  PRT
<213>  Artificial Sequence

<220>
<223>  synthetical protein

<400>  24
Ala Ala Pro Ser Val Thr Leu Phe Pro Pro Ser Ser Glu Glu Leu Gln
1               5                   10                  15
Ala Asn Lys Ala Thr Leu Val Cys Leu Ile Ser Asp Phe Tyr Pro Gly
            20                  25                  30
Ala Val Thr Val Ala Trp Lys Ala Asp Ser Ser Pro Val Lys Ala Gly
        35                  40                  45
Val Glu Thr Thr Thr Pro Ser Lys Gln Ser Asn Asn Lys Tyr Ala Ala
    50                  55                  60
Ser Ser Tyr Leu Ser Leu Thr Pro Glu Gln Trp Lys Ser His Arg Ser
65                  70                  75                  80
Tyr Ser Cys Gln Val Thr His Glu Gly Ser Thr Val Glu Lys Thr Val
                85                  90                  95
Ala Pro Thr Glu Cys Ser
            100

<210>  25
<211>  8
<212>  PRT
<213>  Artificial Sequence

<220>
<223>  synthetical protein

<400>  25
Gly Phe Thr Phe Ser Ser Tyr Trp
1               5

<210>  26
<211>  8
<212>  PRT
<213>  Artificial Sequence

<220>
<223>  synthetical protein

<400>  26
Ile Lys Gln Asp Gly Ser Glu Lys
1               5

<210>  27
<211>  12
<212>  PRT
<213>  Artificial Sequence

<220>
<223>  synthetical protein

<400>  27
Thr Arg Ala Gly Trp Val Arg Gly Ala Phe Asp Ile
1               5                   10

<210>  28
<211>  8
<212>  PRT
<213>  Artificial Sequence

<220>
<223>  synthetical protein

<400>  28
Ser Gly Ser Ile Ala Ser Asn Tyr
1               5

<210>  29
<211>  3
<212>  PRT
<213>  Artificial Sequence

<220>
<223>  synthetical protein

<400>  29
Glu Asp Asn
1

<210>  30
<211>  10
<212>  PRT
<213>  Artificial Sequence

<220>
<223>  synthetical protein

<400>  30
Gln Ser Tyr Asp Ser Ser Asn Leu Trp Val
1               5                   10

<210>  31
<211>  119
<212>  PRT
<213>  Artificial Sequence

<220>
<223>  synthetical protein

<400>  31
Glu Val Gln Leu Val Glu Ser Gly Gly Gly Leu Val Gln Pro Gly Gly
1               5                   10                  15
Ser Leu Arg Leu Ser Cys Ala Ala Ser Gly Phe Thr Phe Ser Ser Tyr
            20                  25                  30
Trp Met Ser Trp Val Arg Gln Ala Pro Gly Lys Gly Leu Glu Trp Val
        35                  40                  45
Ala Asn Ile Lys Gln Asp Gly Ser Glu Lys Tyr Tyr Val Asp Ser Val
    50                  55                  60
Lys Gly Arg Phe Thr Ile Ser Arg Asp Asn Ala Lys Asn Ser Leu Tyr
65                  70                  75                  80
Leu Gln Met Asn Ser Leu Arg Ala Glu Asp Thr Ala Val Tyr Tyr Cys

85                  90                  95
Thr Arg Ala Gly Trp Val Arg Gly Ala Phe Asp Ile Trp Gly Gln Gly
            100                 105                 110
Thr Met Val Thr Val Ser Ser
        115

<210>  32
<211>  115
<212>  PRT
<213>  Artificial Sequence

<220>
<223>  synthetical protein

<400>  32
Asn Phe Met Leu Thr Gln Pro His Ser Val Ser Glu Ser Pro Gly Lys
1               5                   10                  15
Thr Val Thr Ile Ser Cys Thr Gly Ser Ser Gly Ser Ile Ala Ser Asn
            20                  25                  30
Tyr Val Gln Trp Tyr Gln Gln Arg Pro Gly Ser Ala Pro Thr Thr Val
        35                  40                  45
Ile Tyr Glu Asp Asn Gln Arg Pro Ser Gly Val Pro Asp Arg Phe Ser
    50                  55                  60
Gly Ser Ile Asp Ser Ser Ser Asn Ser Ala Ser Leu Thr Ile Ser Gly
65                  70                  75                  80
Leu Lys Thr Glu Asp Glu Ala Asp Tyr Tyr Cys Gln Ser Tyr Asp Ser
                85                  90                  95
Ser Asn Leu Trp Val Phe Gly Gly Gly Thr Lys Leu Thr Val Leu Gly
            100                 105                 110
Gln Pro Lys
        115

<210>  33
<211>  449
<212>  PRT
<213>  Artificial Sequence

<220>
<223>  synthetical protein

<400>  33
Glu Val Gln Leu Val Glu Ser Gly Gly Gly Leu Val Gln Pro Gly Gly
1               5                   10                  15
Ser Leu Arg Leu Ser Cys Ala Ala Ser Gly Phe Thr Phe Ser Ser Tyr
            20                  25                  30
Trp Met Ser Trp Val Arg Gln Ala Pro Gly Lys Gly Leu Glu Trp Val
        35                  40                  45
Ala Asn Ile Lys Gln Asp Gly Ser Glu Lys Tyr Tyr Val Asp Ser Val
    50                  55                  60
Lys Gly Arg Phe Thr Ile Ser Arg Asp Asn Ala Lys Asn Ser Leu Tyr
65                  70                  75                  80
Leu Gln Met Asn Ser Leu Arg Ala Glu Asp Thr Ala Val Tyr Tyr Cys
                85                  90                  95
Thr Arg Ala Gly Trp Val Arg Gly Ala Phe Asp Ile Trp Gly Gln Gly
            100                 105                 110
Thr Met Val Thr Val Ser Ser Ala Ser Thr Lys Gly Pro Ser Val Phe
        115                 120                 125
Pro Leu Ala Pro Ser Ser Lys Ser Thr Ser Gly Gly Thr Ala Ala Leu
    130                 135                 140
Gly Cys Leu Val Lys Asp Tyr Phe Pro Glu Pro Val Thr Val Ser Trp
145                 150                 155                 160
Asn Ser Gly Ala Leu Thr Ser Gly Val His Thr Phe Pro Ala Val Leu
                165                 170                 175

Gln Ser Ser Gly Leu Tyr Ser Leu Ser Ser Val Val Thr Val Pro Ser
            180                 185                 190
Ser Ser Leu Gly Thr Gln Thr Tyr Ile Cys Asn Val Asn His Lys Pro
        195                 200                 205
Ser Asn Thr Lys Val Asp Lys Arg Val Glu Pro Lys Ser Cys Asp Lys
    210                 215                 220
Thr His Thr Cys Pro Pro Cys Pro Ala Pro Glu Leu Leu Gly Gly Pro
225                 230                 235                 240
Ser Val Phe Leu Phe Pro Pro Lys Pro Lys Asp Thr Leu Met Ile Ser
                245                 250                 255
Arg Thr Pro Glu Val Thr Cys Val Val Val Asp Val Ser His Glu Asp
            260                 265                 270
Pro Glu Val Lys Phe Asn Trp Tyr Val Asp Gly Val Glu Val His Asn
        275                 280                 285
Ala Lys Thr Lys Pro Arg Glu Glu Gln Tyr Asn Ser Thr Tyr Arg Val
    290                 295                 300
Val Ser Val Leu Thr Val Leu His Gln Asp Trp Leu Asn Gly Lys Glu
305                 310                 315                 320
Tyr Lys Cys Lys Val Ser Asn Lys Ala Leu Pro Ala Pro Ile Glu Lys
                325                 330                 335
Thr Ile Ser Lys Ala Lys Gly Gln Pro Arg Glu Pro Gln Val Tyr Thr
            340                 345                 350
Leu Pro Pro Ser Arg Asp Glu Leu Thr Lys Asn Gln Val Ser Leu Thr
        355                 360                 365
Cys Leu Val Lys Gly Phe Tyr Pro Ser Asp Ile Ala Val Glu Trp Glu
    370                 375                 380
Ser Asn Gly Gln Pro Glu Asn Asn Tyr Lys Thr Thr Pro Pro Val Leu
385                 390                 395                 400
Asp Ser Asp Gly Ser Phe Phe Leu Tyr Ser Lys Leu Thr Val Asp Lys
                405                 410                 415
Ser Arg Trp Gln Gln Gly Asn Val Phe Ser Cys Ser Val Met His Glu
            420                 425                 430
Ala Leu His Asn His Tyr Thr Gln Lys Ser Leu Ser Leu Ser Pro Gly
        435                 440                 445
Lys


<210>  34
<211>  217
<212>  PRT
<213>  Artificial Sequence

<220>
<223>  synthetical protein

<400>  34
Asn Phe Met Leu Thr Gln Pro His Ser Val Ser Glu Ser Pro Gly Lys
1               5                   10                  15
Thr Val Thr Ile Ser Cys Thr Gly Ser Ser Gly Ser Ile Ala Ser Asn
            20                  25                  30
Tyr Val Gln Trp Tyr Gln Gln Arg Pro Gly Ser Ala Pro Thr Thr Val
        35                  40                  45
Ile Tyr Glu Asp Asn Gln Arg Pro Ser Gly Val Pro Asp Arg Phe Ser
    50                  55                  60
Gly Ser Ile Asp Ser Ser Ser Asn Ser Ala Ser Leu Thr Ile Ser Gly
65                  70                  75                  80
Leu Lys Thr Glu Asp Glu Ala Asp Tyr Tyr Cys Gln Ser Tyr Asp Ser
                85                  90                  95
Ser Asn Leu Trp Val Phe Gly Gly Gly Thr Lys Leu Thr Val Leu Gly
            100                 105                 110
Gln Pro Lys Ala Ala Pro Ser Val Thr Leu Phe Pro Pro Ser Ser Glu
        115                 120                 125
Glu Leu Gln Ala Asn Lys Ala Thr Leu Val Cys Leu Ile Ser Asp Phe

130                 135                 140
Tyr Pro Gly Ala Val Thr Val Ala Trp Lys Ala Asp Ser Ser Pro Val
145                 150                 155                 160
Lys Ala Gly Val Glu Thr Thr Thr Pro Ser Lys Gln Ser Asn Asn Lys
                165                 170                 175
Tyr Ala Ala Ser Ser Tyr Leu Ser Leu Thr Pro Glu Gln Trp Lys Ser
            180                 185                 190
His Arg Ser Tyr Ser Cys Gln Val Thr His Glu Gly Ser Thr Val Glu
        195                 200                 205
Lys Thr Val Ala Pro Thr Glu Cys Ser
    210                 215